# EUROPEAN PATENT APPLICATION

(11) **EP 4 349 776 A1**
(43) Date of publication of application: **10.04.2024**
(21) Application number: 22811683.6
(22) Date of filing: 27.05.2022
(51) Int. Cl.: C01B 25/32, A61L 27/12

(54) **METHOD FOR PREPARING WHITLOCKITE CRYSTALS WITHOUT GENERATION OF HYDROXYAPATITE CRYSTALS**

(30) Priority: 28.05.2021 KR 20210069365
(71) Applicant: H&Bio Co., Ltd., Seongnam-si, Gyeonggi-do 13605 (KR)
(72) Inventor: SHIM, Jung Hee, Seoul 06004 (KR); KIM, Tae Ho, Bucheon-si, Gyeonggi-do 14597 (KR); JO, Hahyeon, Seongnam-si, Gyeonggi-do 13601 (KR)
(74) Representative: Schön, Christoph
(86) International application number: PCT/KR2022/007560
(87) International publication number: WO 2022/250492

(57) **Abstract**

In the present invention, the pH of a solution in a reactor is controlled to be 5 or less so that hydroxyapatite crystals cannot be stably present in order to prevent hydroxyapatite from being generated during preparation of whitlockite. To this end, a basic solution containing calcium ions and magnesium ions was added to an acidic phosphoric acid solution to control that the pH of the solution in the reactor does not exceed 5. As a result, whitlockite having very high purity could be synthesized within a very short time.

## Description

### [Technical Field]

The present application relates to a method for preparing whitlockite crystals.

More particularly, the present application relates to a method for preparing whitlockite crystals without formation of hydroxyapatite crystals in the process of preparing whitlockite crystals.

### [Background Art]

Currently, calcium phosphate compounds are widely used as biocompatible inorganic materials. Calcium phosphate compounds are used as raw materials for products, such as artificial bone, dental restorative composites, bone cement, oral compositions, and fillers, inserted into the body to regenerate or treat human tissue.

As representative calcium phosphate compounds, hydroxyapatite (HAp, Ca₁₀(PO₄)₆(OH)₂) and β-tricalcium phosphate (TCP) (Ca₃(PO₄)₂) are used.

Although artificially synthesized hydroxyapatite has excellent biocompatibility and almost the same chemical properties as bone, it cannot replace natural bone because it is not decomposed *in vivo* due to its high crystallinity.

Since β-TCP is decomposed *in vivo* and induces natural bone growth, many studies are being conducted on single phase β-TCP or biphasic calcium phosphate (BCP) in which β-TCP and hydroxyapatite are mixed. However, according to the related art, it is difficult to synthesize nano-sized β-TCP on a large scale and there is a limit to application of β-TCP to various fields.

To solve these problems, recently, whitlockite, which is a calcium phosphate compound similar to hydroxyapatite and β-TCP, but has a different chemical structure, different components and a different crystal structure, has been studied.

Whitlockite (WH, Ca₁₈Mg₂(HPO₄)₂(PO₄)₁₂), one of the most abundant biomaterials in hard tissue, is present in a high proportion in the body at a young age and in the early stage of biomineralization. This indirectly indicates that whitlockite plays an important role in the development of hard tissue. Particularly, it is known that magnesium contained in whitlockite plays a role in promoting bone formation in surrounding bone tissue in the human body. Based on the fact that whitlockite plays an important role in developing hard tissue, interest in whitlockite as a biocompatible inorganic material replacing conventional hydroxyapatite and β-tricalcium phosphate (β-TCP) is increasing. However, although hydroxyapatite and β-TCP have been actively studied, not much research has been conducted on whitlockite, so the need for research to determine the role and synthesis mechanism of whitlockite is increasing.

However, since the production process of whitlockite is complicated compared to those of hydroxyapatite and β-TCP, not much academic research has been conducted, and it has been difficult to find examples of industrial applications.

To solve the problem that whitlockite synthesis is difficult, a whitlockite preparation method was disclosed by a conventional patent (Korean Patent Publication No. 10-1423982 (July 22, 2014)).

However, the conventional method for preparing whitlockite requires a long production time and high production costs, and is complicated because a desired intermediate product should be formed in an intermediate procedure, and the pH of an aqueous solution needs to be precisely controlled to form high-purity whitlockite.

### [Disclosure]

### [Technical Problem]

To solve the problems of the conventional whitlockite preparation method, the present application is directed to provide a method for preparing whitlockite crystals.

### [Technical Solution]

To solve the problems, the present application provides a method for preparing whitlockite crystals without the formation of hydroxyapatite crystals.

### [Advantageous Effects]

According to the method of the present application, whitlockite crystals can be prepared without the formation of hydroxyapatite crystals.

According to the method of the present application, the time required to prepare whitlockite crystals can be further shortened.

### [Description of Drawings]

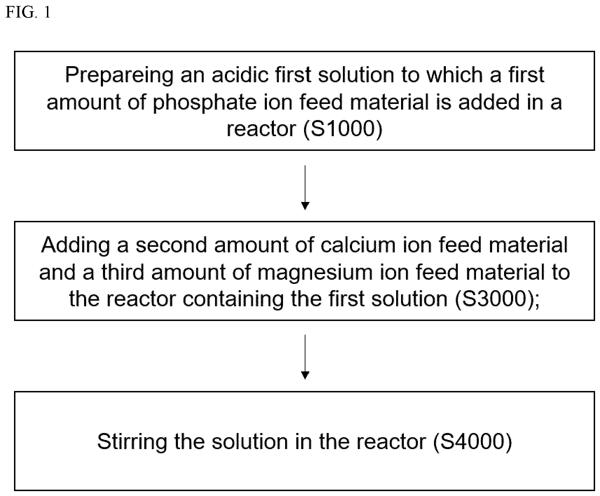

FIG. 1 is a view illustrating a method for preparing whitlockite crystals according to a first embodiment disclosed in the present application.

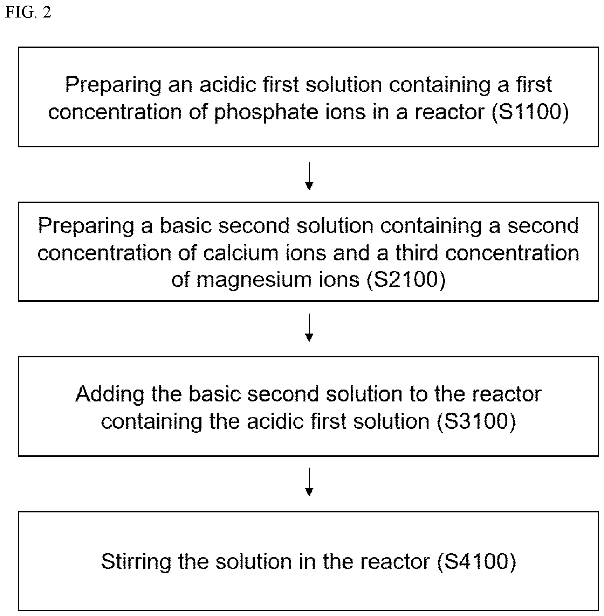

FIG. 2 is a view illustrating a method for preparing whitlockite crystals according to a second embodiment disclosed in the present application.

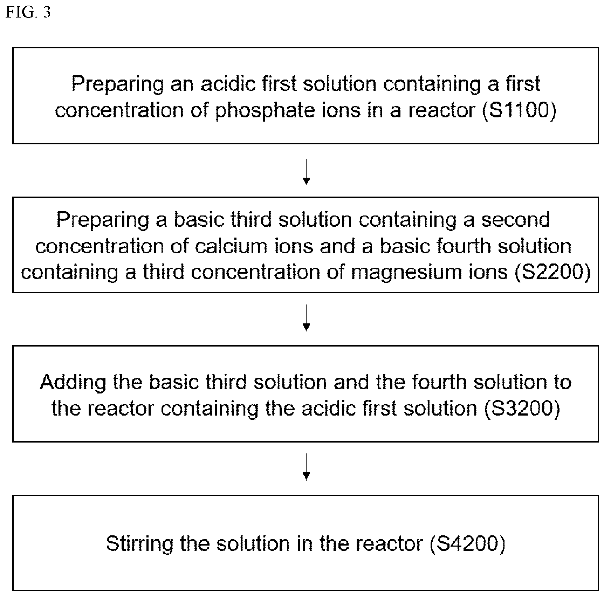

FIG. 3 is a view illustrating a method for preparing whitlockite crystals according to a third embodiment disclosed in the present application.

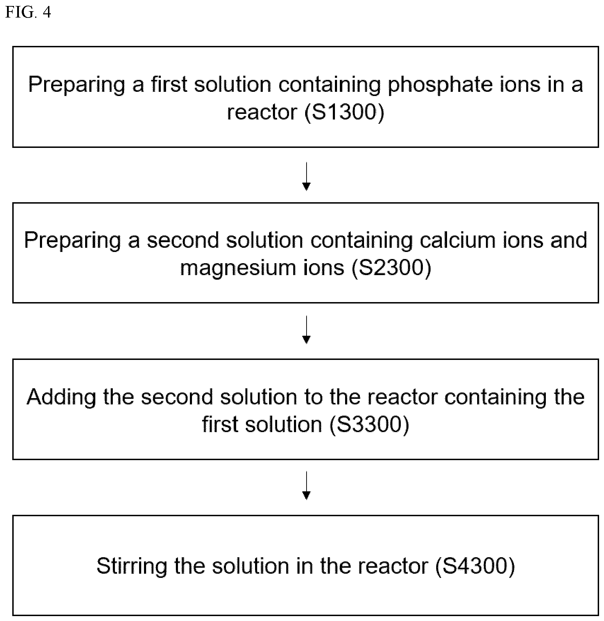

FIG. 4 is a view illustrating a method for preparing whitlockite crystals according to a fourth embodiment disclosed in the present application.
FIG. 5 is a graph showing a pH change of a solution in a reactor in the process of preparing whitlockite crystals when whitlockite crystals are prepared according to Preparation Example 1.
FIG. 6 is a graph showing only the result between 0 to 1200 seconds in the graph of FIG. 5.
FIG. 7 is a graph showing only the result between 0 to 120 seconds in the graph of FIG. 5.
FIG. 8 is a graph of the result obtained by analyzing samples obtained at a specific time point through X-ray diffraction (XRD) in the process of preparing whitlockite crystals when whitlockite crystals are prepared according to Preparation Example 1.
FIG. 9 is a graph of the result obtained by analyzing samples containing high purity whitlockite crystals obtained at 3 hours, 6 hours, and 24 hours after adding a cationic aqueous solution through XRD when whitlockite crystals are prepared according to Preparation Example 1.
FIG. 10 is data on whitlockite crystals obtained at 3 hours, 6 hours, and 24 hours after adding a cationic aqueous solution using a scanning electron microscope (SEM) when whitlockite crystals are prepared according to Preparation Example 1.
FIG. 11 is a view showing the result of XRD analysis of samples obtained at 3 hours, 6 hours, and 24 hours after adding a cationic aqueous solution through XRD when whitlockite crystals are prepared according to Preparation Example 1.
FIG. 12 is a photograph of a product obtained by preparing whitlockite crystals according to a method disclosed in the present application, and washing and drying.
FIG. 13 is an enlarged image of a part of the photograph shown in FIG. 12.
FIG. 14 is a photograph of a product obtained by preparing whitlockite crystals according to a conventional method, and washing and drying the crystals.

### [Modes of the Invention]

According to the present application, a method for preparing whitlockite crystals is provided.

According to one embodiment of the present application, a method for preparing whitlockite crystals without the formation of hydroxyapatite crystals under acidic conditions is provided, the method includes:
(i) preparing a first solution containing phosphate ions in a reactor, wherein the first solution is an acidic solution with a pH of 5 or less;
(ii) preparing a second solution containing calcium ions and magnesium ions, wherein the second solution is a basic solution with a pH of more than 8;
(iii) adding the basic second solution to the reactor including the acidic first solution, wherein a mixed solution of the first solution and the second solution is prepared; and
(iv) stirring the solution in the reactor, wherein a pH of the solution in the reactor is 5 or less.

According to a specific embodiment,
in (iii), the pH of the solution in the reactor may be maintained at 5 or less.

According to a specific embodiment, during the entire process of the method, the pH of the solution in the reactor may be 5 or less.

According to a specific embodiment,
the second solution may be added for a first time period.

Here, according to a specific embodiment, the second solution may be added at a constant rate, and the first time period may be 120 seconds or less.

According to a specific embodiment, in (iii), the solution in the reactor may be stirred.

According to a specific embodiment, in the entire process of the method, hydroxyapatite crystals may not be produced.

According to a specific embodiment, in the entire process of the method, hydroxyapatite crystals may not be produced, and the whitlockite crystals may be
(a) formed directly from the solution in the reactor;
(b) formed by transformation from crystals including calcium atoms other than hydroxyapatite crystals, formed by transformation from crystals including magnesium atoms other than hydroxyapatite crystals, or formed by transformation from crystals including calcium atoms and magnesium atoms other than hydroxyapatite crystals; or
(c) formed by (a) and (b).

According to a specific embodiment, the method may further include drying the resulting product obtained in (iv).

According to a specific embodiment, the method may further include (v) washing the resulting product obtained in (iv); and (vi) drying the resulting product obtained in (v).

According to a specific embodiment, the amount of phosphorus atoms contained in the mixed solution is a first amount, the amount of calcium atoms contained in the mixed solution is a second amount, and the amount of magnesium atoms contained in the mixed solution is a third amount, and
here, any one or more selected from a ratio between the first amount and the second amount, a ratio between the second amount and the third amount, a ratio between the first amount and the third amount are determined by considering one or more of the following:
a ratio of phosphorus atoms, calcium atoms, and magnesium atoms (P: Ca: Mg) constituting whitlockite;
the pH of the mixed solution is 5 or less; and
more than 90 wt% of solids produced through the method is whitlockite crystals.

Here, according to a specific embodiment, the ratio of phosphorus atoms and calcium atoms constituting whitlockite may be 0.78, and the ratio (PICa) of the first amount, which is the amount of phosphorus atoms contained in the mixed solution, and the second amount, which is the amount of calcium atoms contained in the mixed solution, may be greater than 0.78.

According to a specific embodiment, the ratio (Ca/Mg) of the number of moles of calcium atoms contained in the mixed solution and the number of moles of magnesium atoms contained in the mixed solution may be 2.2 to 4.0.

According to one embodiment of the present application, a method for preparing whitlockite crystals under acidic conditions without the formation of hydroxyapatite crystals is provided, the method including:
(i) preparing a first solution containing a first concentration of phosphorous ions in a reactor, wherein the first solution is an acidic solution having a pH of 5 or less;
(ii) preparing a second solution containing a second concentration of calcium ions and a third concentration of magnesium ions, wherein the second solution is a basic solution having a pH of more than 8;
(iii) adding the basic second solution to the reactor including the acidic first solution, wherein a mixed solution in which the first solution and the second solution are mixed is prepared; and
(iv) stirring the solution in the reactor,
wherein the pH of the solution in the reactor is 5 or less.

According to a specific embodiment,
the relationship between the first concentration, the second concentration, and the third concentration is determined so that the mixed solution has a pH of 5 or less, and
based on the determined relationship, the values of the first, second, and third concentrations are selected.

According to a specific embodiment,
in (iii), the pH of the solution in the reactor is maintained at 5 or less.

According to one embodiment of the present application, wherein,
the first concentration is a first molar concentration, the second concentration is a second molar concentration, and the third concentration is a third molar concentration.

According to a specific embodiment,
the second solution is added for a first time period.

Here, according to one embodiment, the second solution may be added at a constant rate, and the first time period may be 120 seconds.

According to a specific embodiment,
the solution in the reactor is stirred.

According to a specific embodiment,
in the entire process of the method, hydroxyapatite crystals are not formed, and
here, the whitlockite crystals produced through the above method may be
   (a) directly precipitated from the solution in the reactor,
   (b) transformed from crystals containing calcium atoms, other than hydroxyapatite crystals, transformed from crystals containing magnesium atoms, other than hydroxyapatite crystals, or transformed from crystals containing calcium and magnesium atoms, other than hydroxyapatite crystals, or
   (c) produced by (a) and (b).

According to a specific embodiment,
the third concentration is 30% to 40% of the second concentration.

According to another embodiment of the present invention, a method for preparing whitlockite crystals under acidic conditions without the formation of hydroxyapatite crystals is provided, the method including:
(i) preparing a first solution containing a first concentration of phosphorous ions in a reactor, wherein the first solution is an acidic solution having a pH of 5 or less;
(ii) preparing a third solution containing a second concentration of calcium ions, and a fourth solution containing a third concentration of magnesium ions, wherein the third solution is a basic solution having a pH of more than 8, and the fourth solution is a basic solution having a pH of more than 8;
(iii) adding the basic third solution and the basic fourth solution to the reactor including the acidic first solution, wherein a mixed solution in which the first solution, the third solution, and the fourth solution are mixed is prepared; and
(iv) stirring the solution in the reactor,
wherein the pH of the solution in the reactor is 5 or less.

### [Disclosure of the Invention]

Hereinafter, specific embodiments of the present application will be described in detail with reference to the drawings. However, the spirit of the invention disclosed in the present application is not limited to the presented embodiments, and those of ordinary skill in the art who understand the spirit of the invention disclosed in the present application will easily suggest other regressive inventions or other embodiments included in the scope of the spirit of the invention disclosed in the present application by adding, changing, or deleting other components within the scope of the same spirit. In addition, the suggested inventions or embodiments are included in the scope of the spirit of the invention disclosed in the present application.

Components with the same function within the scope of the same spirit shown in the drawing of each embodiment are described using the same reference numerals.

When detailed description on the known function or components related to the present invention is determined to unnecessarily obscure the subject matter of the present invention, the detailed description will be omitted. In addition, numbers (e.g., first, second, or the like) used in the description of the specification are merely identifiers for distinguishing one component from another.

The term "approximately" used in the specification refers to an amount, concentration, level, value, number, frequency, percent, dimension, size, weight, or length, which can be changed to 30, 25, 20, 25, 10, 9, 8, 7, 6, 5, 4, 3, 2, or 1% with respect to the reference amount, concentration, level, value, number, frequency, percent, dimension, size, weight, or length.

The ordinal determiners, such as "first," "second," "third," "fourth," and "fifth" etc. used in the present application may be used to distinguish the same terms saperately from each other, if necessary. Accordingly, throughout the specification, terms distinguished as "first" and "second," may not mean the same component, rather, the terms may be used independently for each embodiment or paragraph in which they will be described. For example, in some embodiments, a first amount may refer to an amount of phosphorous atoms, but in another embodiment, a first amount may refer to an amount of magnesium atoms. That is, in each embodiment, referring to the description of the "first amount," the first amount may be interpreted independently for each embodiment. Likewise, in some embodiments, a second amount may refer to an amount of calcium atoms, and in another embodiment, a second amount may refer to an amount of a material for providing calcium ions. As a specific example, in some embodiments, the first amount may be explained as referring to an amount of phosphorous atoms, such as "wherein, the first amount is an amount of phosphorous atoms", but in another embodiment, the first amount may be explained as referring to an amount of a material for providing phosphorous ions, such as "wherein, the first amount is an amount of material for providing phosphorous ions. Not only the n^{th} amount, but also the n^{th} concentration and the n^{th} solution may be independently interpreted for each embodiment or paragraph.

Whitlockite (Ca₁₈Mg₂(HPO₄)₂(PO₄)₁₂) is calcium phosphate containing magnesium atoms and plays an important role in the growth or development of hard tissue as described above. Whitlockite and a conventional method for preparing whitlockite are disclosed in detail in the literature "Phase transformation from hydroxyapatite to the secondary bone mineral, whitlockite"(Jang, Hae Lin, et al. "Phase transformation from hydroxyapatite to the secondary bone mineral, whitlockite." Journal of Materials Chemistry B 3.7 (2015): 1342-1349.)

In the conventional literature, when an acidic atmosphere is created from the beginning of whitlockite preparation and a solution is aged, it is disclosed that high-purity whitlockite cannot be synthesized because calcium phosphate-based compounds such as DCPA and DCPD are produced and remain. On the other hand, since the condition under which hydroxyapatite is stably present is a pH more than 5, when the solution is aged at the pH of more than 5 which is a condition under which hydroxyapatite is formed, due to the presence of hydroxyapatite in the product, it is difficult to synthesize high purity whitlockite. In the conventional literature, to synthesize high purity whitlockite, by adding a material for providing phosphate ions dropwise to a cationic aqueous solution, synthesis was initially carried out in a basic atmosphere to form only a hydroxyapatite phase without forming a DCPA and/or DCPD phase, and after adding all the material for providing phosphate ions, synthesis was carried out in an acidic atmosphere to synthesize high purity whitlockite. In other words, according to the previous art, to produce whitlockite, hydroxyapatite had to be produced first, and then whitlockite was synthesized by a transformation process through the exchange between calcium ions of the produced hydroxyapatite and magnesium ions in the solution.

As described above, whitlockite crystals synthesized by the method disclosed in the conventional literature should be transformed from hydroxyapatite crystals during synthesis. To this end, appropriate pH adjustment was required to ensure that hydroxyapatite crystals could be synthesized in the process of synthesizing whitlockite. More specifically, since hydroxyapatite crystals cannot be stably present at approximately pH 5 or less, the whitlockite synthesis reaction should start from a basic solution, a solution in a reactor should maintain basic conditions for a certain period of time or more while adding raw materials. And after maintaining basic conditions, to transform the produced hydroxyapatite into whitlockite, the solution in the reactor was aged under acidic conditions. Therefore, according to the previous art, the pH of the solution in the reactor needed to be more carefully controlled.

Therefore, to control pH more carefully, conventionally, the addition rate of the acidic solution was suppressed, or the acidic solution was added to the reactor in a dropwise manner. Accordingly, the solution in the reactor was controlled to maintain basic conditions for a certain time period.

However, when whitlockite is synthesized using the above method, the addition rate of the acidic solution is suppressed, so it takes a lot of time to synthesize whitlockite, and the pH should be finely controlled, which is inconvenient.

In addition, to be more industrially used, it is obvious that whitlockite needs to be mass-produced by a process suitable for mass production, but since the addition rate of the acidic solution should be suppressed or a dropwise method should be used, additional and unnecessary costs may be incurred to perform it, and the time required to synthesize whitlockite is also long, which is not suitable for mass production of whitlockite.

Accordingly, the inventors of the present application invented a method for preparing whitlockite crystals in which hydroxyapatite crystals are not produced in the synthesis of whitlockite crystals. According to the method provided by the present application, since whitlockite crystals do not need to be transformed from hydroxyapatite in the process of preparing whitlockite crystals, it is not necessary to carefully control the pH of the solution in the preparation process of whitlockite.

To prevent hydroxyapatite from being produced during the preparation of whitlockite crystals, the inventors of the present application controlled the pH of the solution in the reactor to 5 or less such that hydroxyapatite crystals could not be stably present. When the method disclosed in the present application is used, since it is not necessary for the solution in the reactor to remain under basic conditions for a long time, and there is no need to maintain acidic conditions after the solution in the reactor is necessarily subjected to basic conditions, according to the method of the present application, there is no need to carefully control pH.

To this end, in the method provided in the present application, a method of adding a basic solution containing calcium ions and magnesium ions to an acidic phosphoric acid solution was adopted, and the pH of the solution in the reactor was controlled not to exceed 5 during the addition and aging processes. The method provided in the present application does not use a method of adding or dropping a phosphoric acid solution to calcium ions and magnesium ions. As a result, it was possible to synthesize whitlockite with very high purity in a very short time.

Hereinafter, the method for preparing whitlockite crystals provided by the present application is described in detail.

### Method of preparing whitlockite

To prepare whitlockite crystals, phosphate ions, calcium ions, and magnesium ions need to be mixed and reacted in a solution. During the preparation of whitlockite crystals, phosphate ions are provided by a phosphate ion feed material, calcium ions are provided by a calcium ion feed material, and magnesium ions are provided by a magnesium ion feed material. Usually, the phosphate ion feed material is a material that lowers the pH of a solution when added to the solution, and the calcium ion feed material and the magnesium ion feed material are materials that increase the pH of the solution when added to the solution.

As described above, since hydroxyapatite crystals are not stably present under a condition in which the pH of a solution is 5 or less, in order to prevent hydroxyapatite crystals from being produced during the preparation of whitlockite crystals, the pH of the solution in the reactor should be controlled not to exceed 5.

To control the pH of the solution in the reactor to be 5 or less during the preparation of whitlockite crystals, (1) a cation feed material that increases the pH of the solution should be added to an acidic solution, and the pH of the solution in the reactor should be suppressed to 5 or less during addition. In addition, (2) the pH of a mixed solution, which is a solution obtained after all the raw materials required for preparing whitlockite crystals are added to the solution, should be maintained at 5 or less.

### (1) A cation providing material should be added to an acidic solution.

In order to not produce hydroxyapatite crystals in the preparation of whitlockite crystals, the pH of the solution in the reactor should be maintained at 5 or less, and to this end, a cation feed material should be added to an acidic solution in the method for preparing whitlockite crystals.

The cation feed material includes a calcium ion feed material and a magnesium ion feed material. The acidic solution is a solution to which a phosphate ion feed material is added. That is, to achieve the purpose of the present application, the calcium ion feed material and the magnesium ion feed material should be added to the acidic solution to which the phosphate ion feed material is added.

The process of adding the calcium ion feed material and the magnesium ion feed material to the acidic solution to which the phosphate ion feed material is added may be performed in various ways.

In one example, the process may be performed by adding a solid calcium ion feed material and a solid magnesium ion feed material.

In another example, the process may be performed by adding a basic solution to which a calcium ion feed material and a magnesium ion feed material are added.

In still another example, this process may be performed by adding a first basic solution to which a calcium ion feed material is added and a second basic solution to which a magnesium ion feed material is added.

In yet another example, the process may be performed by adding a basic solution to which a calcium ion feed material is added and adding a solid magnesium ion feed material.

In yet another example, the process may be performed by adding a basic solution to which a magnesium ion feed material is added and adding a solid calcium ion feed material.

The addition of a calcium ion feed material and a magnesium ion feed material may be performed by, but not limited to, a method usually used in the art.

According to a method disclosed in the previous art, since hydroxyapatite crystals need to be produced, the solution in the reactor has to maintain basic conditions for a certain period of time or more while containing some of the raw materials needed to prepare whitlockite crystals. Therefore, in the method disclosed in the previous art, an acidic phosphoric acid solution was added to a basic solution, the addition rate of the phosphoric acid solution was suppressed to allow the solution in the reactor to maintain basic conditions for a certain period of time or more.

According to the method provided by the present application, while adding a calcium ion feed material and a magnesium ion feed material to an acidic solution to which a phosphate ion feed material is added, the pH in the reactor should be maintained at 5 or less. Here, since the purpose of the present application is to prevent hydroxyapatite crystals from being produced, the above purpose may be achieved by preventing the pH of the solution in the reactor from being maintained above 5 for a certain period of time or more. Accordingly, since the case in which the pH of a localized portion of the solution rises above 5 or the case in which the pH in the solution rises above 5 and then decreases to 5 or less before a certain period of time has elapsed during the addition of a calcium ion feed material and a magnesium ion feed material does not affect the achievement of the purpose of the present application, it is obvious that such a case also falls within the scope of the present application.

### (2) The pH of a mixed solution should be maintained at 5 or less.

In order to prevent hydroxyapatite crystals from being produced in the preparation of whitlockite crystals, the pH of the solution in the reaction should be maintained at 5 or less.

Similar to the above description, since the purpose of the present invention is to prevent hydroxyapatite crystals from being produced, it may be achieved by, after adding a cation feed material, preventing the pH of the solution in the reactor, that is, the mixed solution, from being maintained above 5 for a certain period of time or more. Accordingly, since the case in which, after adding the cation feed material, the pH of the solution decreases to 5 or less before a certain period of time has elapsed from a pH above 5 does not affect the achievement of the purpose of the present invention, it is obvious that this case also falls within the scope of the present application.

The pH of the solution in the reactor may vary depending on the temperature of the solution, and to suppress the pH of the solution in the reactor to 5 or less, the temperature of the solution may be considered.

Since the purpose of the present application is not to produce hydroxyapatite crystals, when the temperature of the mixed solution, after adding a cation feed material, is not a temperature suitable for producing hydroxyapatite crystals, the pH of the solution in the reactor may be more than 5. Here, when the temperature in the reactor during the preparation of whitlockite crystals is adjusted to a temperature suitable for producing hydroxyapatite crystals, the pH of the solution in the reactor should be suppressed to 5 or less.

For example, even when the pH of the solution in the reactor is above 5 when the temperature of the mixed solution is not a temperature suitable for producing hydroxyapatite crystals, and then the pH of the solution in the reactor is suppressed to 5 or less while the temperature of the solution increases, this falls within the scope of the present application if the purpose of the present application, which is to prevent formation of hydroxyapatite crystals the preparation of whitlockite crystals, is able to be achieved.

As described above, the formation of hydroxyapatite crystals in the preparation of whitlockite crystals may be affected by the pH of the solution in the reactor, the time period that a solution containing some of the raw materials is maintained under basic conditions, and the temperature of the solution. Accordingly, if the purpose of the present application can be achieved by appropriately adjusting the influencing factors described above, it falls within the scope of the present application.

To maintain the pH of the mixed solution at 5 or less, the amounts of raw materials used in the preparation of whitlockite crystals should be appropriately selected. That is, the amount of each of the phosphate ion feed material, the calcium ion feed material, and the magnesium ion feed material should be determined such that the pH of the mixed solution is 5 or less.

Here, as described above, since the supply of phosphate ions, calcium ions, and magnesium ions may be performed in various ways, the amount of raw materials used may be expressed by being replaced with various units.

In one example, when a solid calcium ion feed material and a solid magnesium ion feed material are added to an acidic solution to which a first amount of phosphate ion feed material is added, a second amount of calcium ion feed material and a third amount of magnesium ion feed material may be added. In this case, to achieve the purpose of the present invention, the first, second, and third amounts are appropriately selected such that the pH of the mixed solution is 5 or less. For example, the first, second and third amounts may each be expressed in units of mass or moles.

Here, the units of mass or moles are units that do not contain information on the volume of the solution. Accordingly, to select appropriate values of ion feed material amounts, the volume of the solution after each ion feed material is added may be considered. In one example, to select an appropriate value of the first amount, the volume of the acidic solution or the volume of the mixed solution may be considered. In one example, to select an appropriate value of the second amount, the volume of the mixed solution may be considered. In one example, to select an appropriate value of the third amount, the volume of the mixed solution may be considered. Even in this case, the first, second, and third amounts should be appropriately selected such that the pH of the mixed solution is 5 or less.

In another example, to the acidic solution to which a first amount of phosphate ion feed material is added, a basic solution to which a second amount of calcium ion feed material and a third amount of magnesium ion feed material are added may be added. In this case, to achieve the purpose of the present invention, the first, second, and third amounts should be appropriately selected such that the pH of the mixed solution is 5 or less.

Here, in the acidic solution, the phosphate ion feed material is ionized into phosphate ions and ions other than phosphate ions, and the acidic solution includes phosphate ions and other ions. In a second solution, a calcium ion feed material and a magnesium ion feed material are ionized, and the second solution includes calcium ions, magnesium ions, and other ions.

That is, in other words, a basic solution containing a second concentration of calcium ions and a third concentration of magnesium ions may be added to an acidic solution containing a first concentration of phosphate ions. In this case, to achieve the purpose of the present invention, the first concentration, the second concentration, and the third concentration should be appropriately selected such that the pH of the mixed solution is 5 or less. In addition, since the volume of the acidic solution and the volume of the basic solution affect the pH of the mixed solution, to select appropriate values of the first concentration, second concentration, and third concentration, the volume of the acidic solution, the volume of the basic solution, and/or the volume of the mixed solution may be considered.

In still another example, to an acidic solution to which a first amount of phosphate ion feed material is added, a first basic solution to which a second amount of calcium ion feed material is added, and a second basic solution to which a third amount of magnesium ion feed material is added may be added. In other words, to an acidic solution containing a first concentration of phosphate ions, a first basic solution containing a second concentration of calcium ions and a second basic solution containing a third concentration of magnesium ions may be added. In this case, as described above, the first concentration, the second concentration, and the third concentration should be appropriately selected such that the pH of the mixed solution is 5 or less. Similar to the above description, to select appropriate values of the first concentration, the second concentration, and the third concentration, the volume of acidic solution, the volume of the first basic solution, the volume of the second basic solution, and/or the volume of the mixed solution may be considered.

Further, since the supply of phosphate ions, calcium ions, and magnesium ions may be performed in various ways, when phosphate ions, calcium ions, and magnesium ions are provided by a method not described above, parameters suitable for a corresponding method may be used to determine the amounts of phosphate ion feed material, calcium ion feed material, and magnesium ion feed material. However, also in this case, to achieve the purpose of the present invention, the amounts of phosphate ion feed material, calcium ion feed material, and magnesium ion feed material used in the preparation of whitlockite should be suitably selected such that the pH of the mixed solution is 5 or less.

Embodiments of the method for preparing whitlockite crystals provided by the present application will be disclosed below. However, as described above, since phosphate ions, calcium ions, and magnesium ions may be provided in various ways in the preparation of whitlockite crystals, the scope of the present application is not limited to the following embodiments. When phosphate ions, calcium ions, or magnesium ions are provided by a method usually used in the art, it falls within the scope of the present application.

### Embodiments

To achieve the purpose of the present application, that is, to prevent hydroxyapatite crystals from being formed in the preparation of whitlockite crystals, the pH of the solution in the reactor should be maintained at 5 or less in the preparation of whitlockite crystals. As described above, to maintain the pH of the solution in the reactor at 5 or less, a calcium ion feed material and a magnesium ion feed material should be added to the acidic solution containing a phosphate ion feed material, and after the addition of all raw materials is completed, the pH of the mixed solution should be maintained at 5 or less.

Hereinafter, in the present application, the following embodiments that can achieve the above-mentioned conditions are provided.

### First embodiment

According to one embodiment of the present application, a method for preparing whitlockite crystals without the formation of hydroxyapatite crystals is provided, the method including:
(i) preparing an acidic first solution to which a first amount of phosphate ion feed material is added in a reactor (S 1000);
(ii) adding a second amount of calcium ion feed material and a third amount of magnesium ion feed material to the reactor containing the first solution (S3000); and
(iii) stirring the solution in the reactor (S4000).

### (i) Preparing acidic first solution to which first amount of phosphate ion feed material is added in reactor (S1000)

The method according to one embodiment of the present application includes `preparing an acidic first solution to which a first amount of phosphate ion feed material is added in a reactor (S 1000).'

Here, according to one embodiment, the phosphate ion feed material may include one or more selected from phosphoric acid, diammonium hydrogen phosphate, ammonium phosphate, and phosphate. Preferably, the phosphate ion feed material may be phosphoric acid.

Here, according to one embodiment, the first amount may be a first mass or first mole. As described above, the units of mass or moles do not include information on the volume of a solution, so the volume of a first solution or the volume of a mixed solution to be prepared may be considered in order to determine the first amount.

A first solution is prepared by adding a first amount of phosphate ion feed material to a predetermined volume of solution. The phosphate ion feed material added to the predetermined volume of solution is ionized into phosphate ions and ions other than the phosphate ions. For example, when phosphoric acid is added to the predetermined volume of solution, the added phosphoric acid is ionized into hydrogen ions (H⁺), dihydrogen phosphate ions (H₂PO₄⁻), hydrogen phosphate ions (HPO₄²⁻), and phosphate ions (PO₄³⁻).

The degree to which the phosphate ion feed material is ionized into phosphate ions in the first solution is changed by the amount of phosphate ion feed material, the degree of ionization of the phosphate ion feed material, the type of compound or ion contained in the first solution, the amount of each compound or ion, and/or the temperature of the solution. Alternatively, the degree to which the phosphate ion feed material is ionized into phosphate ions in the mixed solution to be described below is changed by the degree of ionization of the phosphate ion feed material, the type of compound or ion contained in the first solution, the amount of each compound or ion, and/or the temperature of the solution.

Here, the first solution containing the phosphate ion feed material is an acidic solution. That is, the pH of the first solution is 5 or less. According to one embodiment, the pH of the first solution to which the phosphate ion feed material is added may be approximately 5, 4.5, 4, 3.5, 3, 2.5, 2, 1.5, or 1 or less.

Here, according to one embodiment, the temperature of the first solution may be approximately 10, 15, 20, 25, 30, 35, 40, 45, 50, 55, 60, 65, 70, 75, 80, 85, 90, 95, or 100 °C, but there is no limitation as long as the temperature can achieve the purpose of the present application.

To achieve the purpose of the present application, the first amount should be determined in consideration of a second amount, which is the amount of calcium feed material added in (ii) to be described below, and a third amount, which is the amount of magnesium feed material added in (ii). The relationship between the first, second, and third amounts are disclosed in (ii) below.

### (ii) Adding second amount of calcium ion feed material and third amount of magnesium ion feed material to the reactor containing the first solution (S3000)

As described above, to prevent the formation of hydroxyapatite crystals by maintaining the pH of the solution in the reactor at 5 or less, a calcium ion feed material and a magnesium ion feed material should be added to the reactor containing an acidic solution.

Here, the method according to one embodiment of the present application includes `adding a second amount of calcium ion feed material and a third amount of magnesium ion feed material to the reactor containing the first solution (S3000).'

Here, a mixed solution is prepared by adding a second amount of calcium ion feed material and a third amount of magnesium ion feed material to the reactor containing the first solution.

The calcium ion feed material may include one or more selected from calcium hydroxide, calcium carbonate, calcium nitrate, and calcium acetate. Preferably, the calcium ion feed material may be calcium hydroxide.

The magnesium ion feed material may include one or more selected from magnesium hydroxide, magnesium carbonate, magnesium nitrate, and magnesium acetate. Preferably, the magnesium ion feed material may be magnesium hydroxide.

Here, according to one embodiment, the second amount may be a second mass or a second mole. As described above, the units of mass or moles do not include information on the volume of a solution, so the volume of the mixed solution may be considered in order to determine the second amount.

Here, according to one embodiment, the third amount may be a third mass or a third mole. As described above, the units of mass or mole do not include information on the volume of a solution, so the volume of the mixed solution may be considered in order to determine the third amount.

To synthesize high-purity whitlockite crystals, appropriate values of the second amount, which is the amount of calcium ion feed material and the third amount, which is the amount of magnesium ion feed material may be selected. According to one embodiment of the present application, when the second amount is a second mole and the third amount is a third mole, the third mole may be 25% to 45% of the second mole. According to one embodiment of the present application, the third mole may be 30% to 40% of the second mole. Preferably, according to one embodiment of the present application, the third mole may be 33% to 40% of the second mole.

In the mixed solution to which the calcium ion feed material and the magnesium ion feed material are added, the calcium ion feed material and the magnesium ion feed material are ionized into calcium ions, magnesium ions, and other ions. For example, when calcium hydroxide (Ca(OH)₂) and magnesium hydroxide (Mg(OH)₂) are added, in the mixed solution, the calcium hydroxide is ionized into calcium ions (Ca²⁺) and hydroxide ions (OH⁻), and the magnesium hydroxide is ionized into magnesium ions (Mg²⁺) and hydroxide ions (OH⁻).

In the mixed solution, the degrees to which the calcium ion feed material and the magnesium ion feed material are ionized into calcium ions and magnesium ions are changed by the amount of calcium ion feed material and the amount of magnesium ion feed material, the ionization degrees of the calcium ion material and the magnesium ion feed material, the type of compound or ion contained in the mixed solution, the amount of each compound or ion, and/or the temperature of the solution.

As described above, to achieve the purpose of the present invention, the pH of the solution in the reactor should be maintained at 5 or less. That is, the pH of the mixed solution, which is the solution after adding all of the raw materials used in the preparation of whitlockite crystals should be maintained at 5 or less.

The pH of the mixed solution is determined according to the relationship between the amount of added phosphate ion feed material, the amount of added calcium ion feed material, and the amount of added magnesium ion feed material. Accordingly, the relationship between the first, second, and third amounts should be determined such that the pH of the mixed solution should be 5 or less, and appropriate values of the first, second, and third amounts should be selected based on the determined relationship. Here, since the degree of ionization of phosphate ion feed material, degree of ionization of calcium ion feed material, and degree of ionization of magnesium ion feed material in the mixed solution may affect the pH of the mixed solution, the degree of ionization of each ion feed material may be considered in order to determine the relationship between the first, second, and third amounts such that the pH of the mixed solution is 5 or less.

As described above, since the purpose of the present application is to prevent hydroxyapatite crystals from being formed in the preparation of whitlockite crystals, the case in which the pH rises above 5 in a localized portion of the solution while the calcium ion feed material and the magnesium ion feed material are added or the case in which the pH in the solution rises above 5 and then decreases to 5 or less before a certain period of time has elapsed also falls within the scope of the present application.

According to one embodiment, at the first time point, the addition of a cation feed material to the reactor containing the first solution begins, and the pH of the solution in the reactor immediately after a second period of time has elapsed from the first time point may be 5.5, 5, 4.5, 4, 3.5, 3, 2.5, or 2 or less. Preferably, the pH of the solution in the reactor immediately after the second period of time has elapsed from the first time point is 4.5, 4, 3.5, 3, 2.5, or 2 or less.

Here, according to one embodiment, the solution in the reactor may be stirred while adding the calcium ion feed material and the magnesium ion feed material. During the addition of the calcium ion feed material and the magnesium ion feed material to the solution in the reactor, the materials contained in the solution in the reaction may be properly mixed by stirring.

According to one embodiment of the present application, the calcium ion feed material and the magnesium ion feed material are added to the reactor containing the first solution. Here, the calcium ion feed material and the magnesium ion feed material may be separately added, or added together.

A method of adding a cation feed material, that is, a method of adding a calcium ion feed material and a magnesium ion feed material is not particularly limited, and these materials may be added by a method usually used in the art.

In one example, a solid calcium ion feed material and a solid magnesium ion feed material may be added to the first solution.

In one example, a second solution containing a calcium ion feed material and a magnesium ion feed material may be added to the first solution.

In one example, a third solution containing a calcium ion feed material and a fourth solution containing a magnesium ion feed material may be added to the first solution.

In one example, a third solution containing a calcium ion feed material and a solid magnesium ion feed material may be added to the first solution.

In one example, a fourth solution containing a magnesium ion feed material and a solid calcium ion feed material may be added to the first solution.

According to one embodiment of the present application, a calcium ion feed material may be added to the solution contained in the reactor for a first period of time. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. In a specific embodiment, the first period of time may be approximately 180, 160, 140, 120, 100, 80, 60, 50, 40, 30, 20, or 10 seconds. In a specific embodiment, the first period of time may be 180, 160, 140, 120, 100, 80, 60, 50, 40, 30, 20, or 10 seconds or less.

According to one embodiment of the present application, a magnesium ion feed material may be added to the solution in the reactor for a second period of time. According to one embodiment, the second period of time may be 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the second period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. According to a specific embodiment, the second period of time may be approximately 180, 160, 140, 120, 100, 80, 60, 50, 40, 30, 20, or 10 seconds. In a specific embodiment, the second period of time may be 180, 160, 140, 120, 100, 80, 60, 50, 40, 30, 20, or 10 seconds or less.

### (iii) Stirring the solution in the reactor (S4000)

According to one embodiment of the present application, the method for preparing whitlockite crystals includes `(iv) stirring the solution in the reactor (S4000).' In this process, to prepare whitlockite crystals, a process of aging the solution may be included.

As described above, the solution in the reactor should be maintained under acidic conditions. According to one embodiment, the pH of the solution in the reactor may be 5.5, 5, 4.5, 4, 3.5, 3, 2.5, or 2 or less. Preferably, the pH of the solution in the reactor may be 4.5, 4, 3.5, 3, or 2.5 or less.

According to the method disclosed in the previous art, since hydroxyapatite crystals need to be produced, the solution in the reactor has to maintain basic conditions for a certain period of time or more while containing some of raw materials needed to prepare whitlockite crystals. Accordingly, in the method disclosed in the previous art, an acidic phosphoric acid solution is added to a basic solution, and the addition rate of the phosphoric acid solution is suppressed to allow the solution in the reaction to maintain basic conditions for a certain period of time or more.

On the other hand, according to the method provided by the present application, whitlockite crystals are prepared without the formation of hydroxyapatite crystals in the preparation of whitlockite crystals. Therefore, by the method provided by the present application, the solution in the reactor does not need to maintain basic conditions for a certain period of time or more while containing raw materials.

According to one embodiment, the addition of a cation feed material to the reactor containing the first solution is terminated at a second time point, and the pH of the solution in the reactor immediately after a third period of time has elapsed from the second time point may be 5.5, 5, 4.5, 4, 3.5, 3, 2.5, or 2 or less. Preferably, the pH of the solution in the reactor immediately after the third period of time has elapsed from the second time point is 4.5, 4, 3.5, 3, 2.5, or 2 or less.

To prepare whitlockite crystals, after terminating the addition of a cation feed material, the material contained in the reactor may be properly mixed by stirring.

According to one embodiment, here, the solution in the reactor may be stirred at approximately 30, 35, 40, 45, 50, 55, 60, 65, 70, 75, 80, 85, 90, 95, or 100 °C. According to one embodiment, the solution in the reactor may be stirred at 20 °C to 100 °C. Preferably, the solution in the reactor may be stirred at 50 °C to 90 °C. More preferably, the solution in the reactor may be stirred at 70 °C to 90 °C.

According to one embodiment, (iii) stirring the solution in the reactor (S4000) may be performed for approximately 1, 2, 3, 4, 5, 6, 7, 8, 9, 10, 12, 14, 16, 18, 20, 22, 24, 28, 32, 36, 40, 45, or 50 hours, or for longer than these times values. In a specific embodiment, (iii) stirring the solution in the reaction (S4000) may be performed for approximately 6, 7, 8, 9, 10, 12, 14, 16, 18, 20, 22, or 24 hours, or for longer than these values of time.

According to one embodiment, materials for adjusting the pH of the solution may be further added such that the pH of the solution in the reactor is the above-mentioned pH value.

As described above, the calcium ion feed material and the magnesium ion feed material may be added to an acidic first solution in various ways. Hereinafter, an example in which the addition of a cation feed material is performed by adding a basic solution containing a calcium ion feed material and a magnesium ion feed material is disclosed.

### (iv) Steps that can be selectively included

Hereinafter, steps that can be selectively included in the method for preparing whitlockite crystals according to the first embodiment will be described. For example, the method for preparing whitlockite crystals according to the first embodiment may selectively further include washing the resulting product, drying the resulting product, and grinding the resulting product. Here, the washing may be performed to remove ions remaining in the solution after the reaction is completed, but the present invention is not limited thereto.

In one embodiment, the method for preparing whitlockite crystals according to the first embodiment may further include washing the resulting product obtained in (iii).

In one embodiment, the method for preparing whitlockite crystals according to the first embodiment may further include drying the resulting product obtained in (iii).

In one embodiment, the method for preparing whitlockite crystals according to the first embodiment may further include (iv) washing the resulting product obtained in (iii); and (v) drying the resulting product obtained in (iv).

In one embodiment, the washing may be performed by a method of filtering solids in the resulting product, but the present invention is not limited thereto. For example, the washing may be performed by a method of filtering solids in the resulting product using a filter press and removing a liquid in the resulting product or ions remaining in the liquid.

Through the method according to the present embodiment (first embodiment), whitlockite may be prepared. When crystals prepared by the method according to this embodiment are analyzed by XRD, more than 90 wt% of the prepared crystals may be identified as whitlockite crystals. In one embodiment, the XRD analysis results of the crystals prepared by the method according to this embodiment may show that 90, 91, 92, 93, 94, 95, 96, 97, 98, 99, or 99.9 wt% or more of the prepared crystals are whitlockite crystals. In a specific example, the XRD analysis results of the crystals prepared by the method according to this embodiment may show that 99 wt% or 99.9 wt% or more of the prepared crystals are whitlockite crystals. In a specific embodiment, the XRD analysis results of the crystals prepared by the method according to this embodiment may show that 100 wt% of the prepared crystals are whitlockite crystals.

In one embodiment, 90, 91, 92, 93, 94, 95, 96, 97, 98, 99, or 99.9 wt% or more of the solids produced by the method according to this embodiment may be whitlockite crystals. In a specific embodiment, 99 or 99.9 wt% or more of the solids produced by the method according to this embodiment may be whitlockite crystals. In a specific embodiment, all of the solids produced according to this embodiment may be whitlockite crystals.

### Second embodiment

The addition of a cation feed material may be performed through various methods. According to one embodiment, the addition of a cation feed material may be performed by a method of adding a second solution containing a calcium ion feed material and a magnesium ion feed material.

A phosphate ion feed material is ionized into phosphate ions and other ions in a solution, and the calcium ion feed material and the magnesium ion feed material are ionized into calcium ions, magnesium ions, and other ions in the solution.

In the present application, a method for preparing whitlockite crystals without the formation of hydroxyapatite crystals is provided, the method including:
(i) preparing an acidic first solution containing a first concentration of phosphate ions in a reactor (S 1100);
(ii) preparing a basic second solution containing a second concentration of calcium ions and a third concentration of magnesium ions (S2100);
(iii) adding the basic second solution to the reactor containing the acidic first solution (S3100); and
(iv) stirring the solution in the reactor (S4100).

### (i) Preparing acidic first solution containing first concentration of phosphate ions in reactor (S1100)

The method according to one embodiment of the present application includes `preparing an acidic first solution containing a first concentration of phosphate ions in a reactor (S1100).'

Here, according to one embodiment, a first concentration may be a first molar concentration, but the present invention is not limited thereto, and may be expressed in units of concentration usually used in the art.

A first solution is prepared by adding a phosphate ion feed material to a predetermined volume of solution. The phosphate ion feed material added to the predetermined volume of solution is ionized into phosphate ions and ions other than the phosphate ions. Accordingly, the first solution may include a first concentration of phosphate ions. For example, the first solution containing the first concentration of phosphate ions may be prepared by adding a first amount of phosphoric acid to a predetermined volume of solution.

In the first solution, the degree to which the phosphate ion feed material is ionized into phosphate ions is changed by the amount of phosphate ion feed material, the degree of ionization of the phosphate ion feed material, the type of compound or ion contained in the first solution, the amount of each compound or ion, and/or the temperature of the solution. Alternatively, the degree to which the phosphate ion feed material is ionized into phosphate ions in the mixed solution to be described below is changed by the amount of phosphate ion feed material, the degree of ionization of the phosphate ion feed material, the type of compound or ion contained in the first solution, the amount of each compound or ion, and/or the temperature of the solution.

Here, the first solution containing the first concentration of phosphate ions is an acidic solution. That is, the pH of the first solution is 5 or less. According to one embodiment, the pH of the first solution may be approximately 5, 4.5, 4, 3.5, 3, 2.5, 2, 1.5, or 1 or less.

Here, according to one embodiment, the temperature of the first solution may be approximately 10, 15, 20, 30, 35, 40, 45, 50, 55, 60, 65, 70, 75, 80, 85, 90, 95 or 100 °C, but there is no limitation as long as the temperature can achieve the purpose of the present application.

To achieve the purpose of the present application, the first concentration should be determined in consideration of a second concentration, which is a concentration of calcium ions added in (iii) to be described below, and a third concentration, which is a concentration of magnesium ions added in (iii). Therefore, the relationship between the first, second, and third amounts are disclosed in (iii) below.

### (ii) Preparing basic second solution containing second concentration of calcium ions and third concentration of magnesium ions (S2100)

Subsequently, the method includes `preparing a basic second solution containing a second concentration of calcium ions and a third concentration of magnesium ions (S2100).'

Here, according to one embodiment, the second concentration may be a second molar concentration, and the third concentration may be a third molar concentration. However, these concentrations are not limited thereto, and may be expressed in units of concentration usually used in the art.

A second solution may be prepared by adding a second amount of calcium ion feed material and a third amount of magnesium ion feed material to a predetermined volume of solution. The added calcium ion feed material and magnesium ion feed material are ionized into calcium ions, magnesium ions, and other ions. Accordingly, the second solution may include a second concentration of calcium ions and a third concentration of magnesium ions. For example, the second solution containing the second concentration of calcium ions and the third concentration of magnesium ions may be prepared by adding a second amount of calcium hydroxide and a third amount of magnesium hydroxide to a predetermined volume of solution.

In the second solution, the degrees to which the calcium ion feed material and the magnesium ion feed material are ionized into calcium ions and magnesium ions are changed by the amount of calcium ion feed material and the amount of magnesium ion feed material, the ionization degrees of the calcium ion material and the magnesium ion feed material, the type of compound or ion contained in the second solution, the amount of each compound or ion, and/or the temperature of the solution. In addition, in a mixed solution to be described below, the degrees to which the calcium ion feed material and the magnesium ion feed material are ionized into calcium ions and magnesium ions is changed by the amount of calcium ion feed material and the amount of magnesium ion feed material, the ionization degrees of the calcium ion material and the magnesium ion feed material, the type of compound or ion contained in the second solution, the amount of each compound or ion, and/or the temperature of the solution.

Here, the second solution containing a second concentration of calcium ions and a third concentration of magnesium ions is a basic solution. That is, the pH of the second solution is more than 7. According to one embodiment, the pH of the second solution may be 8, 8.5, 9, 9.5, 10, 10.5, 11, 11.5, 12, 12.5, or 13 or more.

To synthesize high-purity whitlockite crystals, appropriate values of the concentration of calcium ions and the concentration of magnesium ions should be selected. According to one embodiment of the present application, in the second solution, a third concentration, which is the concentration of magnesium ions may be 25% to 45% of the second concentration, which is the concentration of calcium ions. According to one embodiment of the present application, the third concentration may be 30% to 40% of the second concentration. Preferably, according to one embodiment of the present application, the third concentration is 33% to 40% of the second concentration.

Here, according to one embodiment, the temperature of the second solution may be approximately 10, 15, 20, 25, 30, 35, 40, 45, 50, 55, 60, 65, 70, 75, 80, 85, 90, 95, or 100 °C, and there is no limitation as long as it can achieve the purpose of the present application.

### (iii) Adding basic second solution to the reactor containing the acidic first solution (S3100)

As described above, to prevent the formation of hydroxyapatite crystals by maintaining the pH of the solution in the reactor at 5 or less, a basic solution should be added to the reactor containing the acidic solution.

Accordingly, the method according to one embodiment of the present application includes `adding the basic second solution to the reactor containing the acidic first solution (S3 100).'

Here, the first solution includes a first concentration of phosphate ions, and the second solution includes a second concentration of calcium ions and a third concentration of magnesium ions.

Here, the second solution is added to the reactor containing the first solution, thereby preparing a mixed solution in which the first solution and the second solution are mixed.

As described above, to achieve the purpose of the present invention, the pH of the solution in the reactor should be maintained at 5 or less. That is, the pH of the mixed solution in which the first solution and the second solution are mixed should be maintained at 5 or less.

Here, the concentration of phosphate ions contained in the first solution, the concentrations of calcium ions and magnesium ions contained in the second solution affect the pH of the mixed solution. Accordingly, the relationship between the first concentration, the second concentration, and the third concentration should be determined such that the pH of the mixed solution is 5 or less, and based on the determined relationship, appropriate values of the first concentration, the second concentration, and the third concentration should be selected.

Since the degree to which the phosphate ion feed material added to prepare the first solution in (i) is ionized in the mixed solution, and the degrees to which the calcium ion feed material and the magnesium ion feed material added to prepare the second solution in (ii) are ionized in the mixed solution may affect the pH of the mixed solution, the relationship between the first concentration, the second concentration, and the third concentration may be determined such that the pH of the mixed solution is 5 or less by considering the degrees of ionization for each ion feed material in the mixed solution.

The volume of the first solution and the volume of the second solution may also affect the pH of the mixed solution. Accordingly, the relationship between the first concentration, the second concentration, and the third concentration may be determined such that the pH of the mixed solution is 5 or less by considering the volume of the first solution and the volume of the second solution.

As described above, since the purpose of the present application is to prevent hydroxyapatite crystals from being formed in the preparation of whitlockite crystals, the case in which the pH of a localized portion of the solution in the reactor rises above 5 while adding the second solution to the first solution or the case in which the pH in the solution rises above 5 and then decreases to 5 or less before a certain period of time has elapsed also falls within the scope of the present application.

According to one embodiment, the addition of the second solution to the reactor containing the first solution starts at the first time point, and the pH of the solution in the reactor immediately after a second period of time has elapsed from the first time point may be 5.5, 5, 4.5, 4, 3.5, 3, 2.5, or 2 or less. Preferably, the pH of the solution in the reactor immediately after the second period of time has elapsed from the first time point is 4.5, 4, 3.5, 3, 2.5, or 2 or less.

The volumes of the first solution and the second solution are not particularly limited. For example, the volume of the first solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. For example, the volume of the second solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. In one embodiment, the volume ratio of the first and second solutions (the volume of the first solution / the volume of the second solution) may be approximately 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.2, 3.4, 3.6, 3.8, 4, 4.5, 5, 5.5, 6, 6.5, 7, 7.5, 8, 9, 10, 20, 30, 40, 50, or 100, but the present invention is not limited thereto.

According to one embodiment, the second solution may be added to the reactor containing the first solution through pouring. According to one embodiment, the second solution may be added to the reactor containing the first solution for a certain period of time. According to one embodiment, the second solution may be added to the reactor containing the first solution at a certain rate for a certain period of time. The second solution may be added by a method usually used in the art.

According to the method disclosed in the conventional literature, since the solution in the reactor has to maintain basic conditions for a certain period of time or more, an acidic solution may be added while suppressing the addition rate thereof, or added dropwise. As disclosed in the conventional literature, the addition of the acidic solution is performed for 40 minutes.

On the other hand, according to the method for preparing whitlockite crystals according to one embodiment of the present application, there is no need to suppress the addition rate of the basic second solution. Accordingly, by the method disclosed in the present application, the time required to prepare whitlockite crystals is reduced by shortening the addition time of the solution.

According to one embodiment of the present application, the second solution may be added to the reactor containing the first solution for a first period of time. According to one embodiment, the first period of time may be approximately 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. In a specific embodiment, the first period of time may be approximately 180, 160, 140, 120, 100, 80, 60, 50, 40, 30, 20, or 10 seconds. In a specific embodiment, the first period of time may be 180, 160, 140, 120, 100, 80, 60, 50, 40, 30, 20, or 10 seconds or less.

Here, according to one embodiment, during the process of adding the second solution, the solution in the reactor may be stirred. During the addition of the second solution, the materials contained in the solution in the reactor may be properly mixed by stirring.

### (iv) Stirring the solution in the reactor (S4100)

The method for preparing whitlockite crystals according to one embodiment of the present application includes `(iv) stirring the solution in the reactor (S4100).' This process may include a process of aging the solution to prepare whitlockite crystals.

As described above, the solution in the reactor should be maintained under acidic conditions. According to one embodiment, the pH of the solution in the reactor may be 5.5, 5, 4.5, 4, 3.5, 3, 2.5, or 2 or less. Preferably, the pH of the solution in the reactor is 4.5, 4, 3.5, 3, or 2.5 or less.

As described above, when whitlockite crystals are synthesized by the method disclosed in the previous art, hydroxyapatite crystals need to be produced, so the solution in the reactor has to maintain basic conditions for a certain period of time or more while containing some of the raw materials required for preparing whitlockite crystals. Accordingly, in the method disclosed in the previous art, an acidic phosphoric acid solution is added to the basic solution, and the addition rate of the acidic phosphoric acid solution is suppressed such that the solution in the reactor maintains basic conditions for a certain period of time or more.

On the other hand, according to the method provided by the present application, whitlockite crystals are prepared without the formation of hydroxyapatite crystals in the preparation of whitlockite crystals. Accordingly, by the method provided by the present application, the solution in the reactor does not need to maintain basic conditions for a certain period of time or more while containing the raw materials.

Since the purpose of the present application is to prevent the formation of hydroxyapatite crystals, according to one embodiment, after adding the second solution, the described purpose may be achieved by preventing the pH of the solution in the reactor from being maintained more than 5 for a certain period of time or more.

According to one embodiment of the present application, the addition of the second solution is terminated at the second time point, and the pH of the solution in the reactor immediately after a third period of time has elapsed from the second time point may be 5.5, 5, 4.5, 4, 3.5, 3, 2.5, or 2 or less. Preferably, the pH of the solution in the reactor immediately after a third period of time has elapsed from the second time point may be 4.5, 4, 3.5, 3, 2.5, or 2 or less.

To prepare whitlockite crystals, after terminating the addition of the second solution, the materials contained in the solution in the reactor may be properly mixed through stirring.

According to one embodiment, here, the solution in the reactor may be stirred at approximately 30, 35, 40, 45, 50, 55, 60, 65, 70, 75, 80, 85, 90, 95, or 100 °C. According to one embodiment, the solution in the reactor may be stirred at 20 to 100 °C. Preferably, the solution in the reactor may be stirred at 50 °C to 90 °C. More preferably, the solution in the reactor may be stirred at 70 °C to 90 °C.

According to one embodiment, (iv) stirring the solution in the reactor (S4100) may be performed for approximately 1, 2, 3, 4, 5, 6, 7, 8, 9, 10, 12, 14, 16, 18, 20, 22, 24, 28, 32, 36, 40, 45, or 50 hours, or for longer than these times values. In a specific embodiment, (iv) stirring the solution in the reactor (S4100) may be performed for approximately 6, 7, 8, 9, 10, 12, 14, 16, 18, 20, 22, or 24 hours, or for longer than these times values.

According to one embodiment, materials that can control the pH of the solution may be further added such that the pH of the solution in the reactor becomes the above-described pH value.

### (v) Steps that can be selectively included

Hereinafter, steps that can be selectively included in the method for preparing whitlockite crystals according to the second embodiment will be described. For example, the method for preparing whitlockite crystals according to the second embodiment may selectively further include washing the resulting product, drying the resulting product, and grinding the resulting product. Here, the washing may be performed to remove ions remaining in the solution after the reaction is completed, but the present invention is not limited thereto.

In one embodiment, the method for preparing whitlockite crystals according to the second embodiment may further include washing the resulting product obtained in (iv).

In one embodiment, the method for preparing whitlockite crystals according to the second embodiment may further include drying the resulting product obtained in (iv).

In one embodiment, the method for preparing whitlockite crystals according to the second embodiment may further include (v) washing the resulting product obtained in (iv); and (vi) drying the resulting product obtained in (v).

In one embodiment, the washing may be performed by a method of filtering solids in the resulting product, but the present invention is not limited thereto. For example, the washing may be performed by a method of filtering solids in the resulting product using a filter press and removing a liquid in the resulting product or ions remaining in the liquid.

Through the method according to the present embodiment (second embodiment), whitlockite may be prepared. When crystals prepared by the method according to this embodiment are analyzed by XRD, more than 90 wt% of the prepared crystals may be identified as whitlockite crystals. In one embodiment, the XRD analysis results of the crystals prepared by the method according to this embodiment may show that 90, 91, 92, 93, 94, 95, 96, 97, 98, 99, or 99.9 wt% or more of the prepared crystals are whitlockite crystals. In a specific example, the XRD analysis results of the crystals prepared by the method according to this embodiment may show that 99 wt% or 99.9 wt% or more of the prepared crystals are whitlockite crystals. In a specific embodiment, the XRD analysis results of the crystals prepared by the method according to this embodiment may show that 100 wt% of the prepared crystals are whitlockite crystals.

In one embodiment, 90, 91, 92, 93, 94, 95, 96, 97, 98, 99, or 99.9 wt% or more of the solids produced by the method according to this embodiment may be whitlockite crystals. In a specific embodiment, 99 or 99.9 wt% or more of the solids produced by the method according to this embodiment may be whitlockite crystals. In a specific embodiment, all of the solids produced according to this embodiment may be whitlockite crystals.

### Third Embodiment

According to another embodiment of the present application, a method for preparing whitlockite crystals without the formation of hydroxyapatite crystals is provided, the method including:
(i) preparing an acidic first solution containing a first concentration of phosphate ions in a reactor (S 1100);
(ii) preparing a basic third solution containing a second concentration of calcium ions and a basic fourth solution containing a third concentration of magnesium ions (S2200);
(iii) adding the basic third solution and the fourth solution to the reactor containing the acidic first solution (S3200); and
(iv) stirring the solution in the reactor (S4200).

Hereinafter, the third embodiment will be described in detail. The same content as in the above-described embodiments will be omitted.

### (ii) Preparing basic third solution containing second concentration of calcium ions and basic fourth solution containing third concentration of magnesium ions (S2200);

According to one embodiment, the method may include `preparing a basic third solution containing a second concentration of calcium ions and a basic fourth solution containing a third concentration of magnesium ions (S2200).'

According to one embodiment, a third solution may be prepared by the following method: A third solution may be prepared by adding a calcium ion feed material to a predetermined volume of solution. The added calcium ion feed material is ionized into calcium ions and other ions. Therefore, the third solution may include a second concentration of calcium ions.

Here, the third solution containing the second concentration of calcium ions is a basic solution. That is, the pH of the third solution is more than 7. According to one embodiment, the pH of the third solution may be 8, 8.5, 9, 9.5, 10, 10.5, 11, 11.5, 12, 12.5, or 13 or more.

Here, according to one embodiment, the temperature of the third solution may be approximately 10, 15, 20, 25, 30, 35, 40, 45, 50, 55, 60, 65, 70, 75, 80, 85, 90, 95, or 100 °C, but there is no limitation as long as the temperature can achieve the purpose of the present application.

According to one embodiment, the fourth solution may be prepared by the following method: A fourth solution may be prepared by adding a magnesium ion feed material to a predetermined volume of solution. The added magnesium ion feed material is ionized into magnesium ions and other ions. Accordingly, the fourth solution may include a third concentration of magnesium ions.

Here, the fourth solution containing the third concentration of magnesium ions is a basic solution. That is, the pH of the fourth solution is more than 7. According to one embodiment, the pH of the fourth solution may be 8, 8.5, 9, 9.5, 10, 10.5, 11, 11.5, 12, 12.5, or 13 or more.

Here, according to one embodiment, the temperature of the fourth solution may be approximately 10, 15, 20, 25, 30, 35, 40, 45, 50, 55, 60, 65, 70, 75, 80, 85, 90, 95, or 100 °C, but there is no limitation as long as the temperature can achieve the purpose of the present application.

### (iii) Adding the basic third solution and the fourth solution to the reactor containing the acidic first solution (S3200)

According to one embodiment, the method may include `adding the basic third solution and the fourth solution to the reactor containing the acidic first solution (S3200).'

Here, a mixed solution in which the first, third, and fourth solutions are mixed is prepared by adding the third solution and the fourth solution to the reactor containing the first solution.

As described above, the pH of the mixed solution should be maintained at 5 or less.

Here, the concentration of phosphate ions contained in the first solution, the concentration of calcium ions contained in the third solution, and the concentration of magnesium ions contained in the fourth solution affect the pH of the mixed solution. Accordingly, the relationship between the first concentration, the second concentration, and the third concentration should be determined such that the pH of the mixed solution is 5 or less, and based on the determined relationship, appropriate values of the first concentration, the second concentration, and the third concentration should be selected.

Since the degree to which the phosphate ion feed material added to prepare the first solution in (i) is ionized in the mixed solution, and the degrees to which the calcium ion feed material added to prepare the third solution and the magnesium ion feed material added to prepare the fourth solution in (ii) are ionized in the mixed solution may affect the pH of the mixed solution, the relationship between the first concentration, the second concentration, and the third concentration may be determined such that the pH of the mixed solution is 5 or less by considering the degrees of ionization of each ion in the mixed solution.

The volume of the first solution, the volume of the third solution, and the volume of the fourth solution may also affect the pH of the mixed solution. Accordingly, the relationship between the first concentration, the second concentration, and the third concentration may be determined such that the pH of the mixed solution is 5 or less by considering the volume of the first solution, the volume of the third solution, and the volume of the fourth solution.

The case in which the pH of a localized portion of the solution in the reactor rises above 5 while adding the third solution and the fourth solution to the first solution or the case in which the pH in the solution rises above 5 and then decreases to 5 or less before a certain period of time has elapsed also falls within the scope of the present application.

As described in the second embodiment, the volume of the solutions used in the method of the present application is not particularly limited. For example, the volume of the first solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. For example, the volume of the third solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. For example, the volume of the fourth solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto.

According to one embodiment, the third solution may be added to the reactor containing the first solution by pouring. According to one embodiment, the third solution may be added to the reactor containing the first solution for a certain period of time. According to one embodiment, the third solution may be added to the reactor containing the first solution at a certain rate for a certain period of time. The third solution may be added by a method usually used in the art.

According to one embodiment, the fourth solution may be added to the reactor containing the first solution by pouring. According to one embodiment, the fourth solution may be added to the reactor containing the first solution for a certain period of time. According to one embodiment, the fourth solution may be added to the reactor containing the first solution at a certain rate for a certain period of time. The fourth solution may be added by a method usually used in the art.

According to one embodiment, the third solution may be added to the reactor containing the first solution for a first period of time. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. In a specific embodiment, the first period of time may be approximately 180, 160, 140, 120, 100, 80, 60, 50, 40, 30, 20, or 10 seconds. In a specific embodiment, the first period of time may be 180, 160, 140, 120, 100, 80, 60, 50, 40, 30, 20, or 10 seconds or less.

According to one embodiment, the fourth solution may be added to the reactor containing the first solution for a second period of time. According to one embodiment, the second period of time may be 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the second period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. In a specific embodiment, the second period of time may be approximately 180, 160, 140, 120, 100, 80, 60, 50, 40, 30, 20, or 10 seconds. In a specific embodiment, the second period of time may be 180, 160, 140, 120, 100, 80, 60, 50, 40, 30, 20, or 10 seconds or less.

Here, according to one embodiment, during the process of adding the third solution and the fourth solution, the solution in the reactor may be stirred. During the addition of the third solution and the fourth solution, the materials contained in the solution in the reactor may be properly mixed by stirring.

### (iv) Stirring the solution in the reactor (S4200)

The method for preparing whitlockite crystals according to one embodiment of the present application includes `(iv) stirring the solution in the reactor (S4200).' This process may include aging the solution to prepare whitlockite crystals.

As described above, the solution in the reactor should be maintained under acidic conditions. According to one embodiment, the pH of the solution in the reactor may be 5.5, 5, 4.5, 4, 3.5, 3, 2.5, or 2 or less. Preferably, the pH of the solution in the reactor may be 4.5, 4, 3.5, 3, or 2.5 or less.

When whitlockite crystals are synthesized by the method disclosed in the previous art, hydroxyapatite crystals need to be produced, so the solution in the reactor has to maintain basic conditions for a certain period of time or more while containing some of the raw materials required for preparing whitlockite crystals. Accordingly, in the method disclosed in the previous art, an acidic phosphoric acid solution is added to the basic solution, and the addition rate of the phosphoric acid solution is suppressed such that the solution in the reactor maintains basic conditions for a certain period of time or more.

On the other hand, according to the method provided by the present application, whitlockite crystals are prepared without the formation of hydroxyapatite crystals during the preparation of whitlockite crystals. Accordingly, by the method provided by the present application, the solution in the reactor does not need to maintain basic conditions for a certain period of time or more while containing the raw materials.

Since the purpose of the present application is to prevent the formation of hydroxyapatite crystals, according to one embodiment, after adding the third solution and the fourth solution, the described purpose may be achieved by preventing the pH of the solution in the reactor from being maintained more than 5 for a certain period of time or more.

To prepare whitlockite crystals, after terminating the addition of the second solution, the materials contained in the solution in the reactor may be properly mixed by stirring.

According to one embodiment, here, the solution in the reactor may be stirred at approximately 30, 35, 40, 45, 50, 55, 60, 65, 70, 75, 80, 85, 90, 95, or 100 °C. According to one embodiment, the solution in the reactor may be stirred at 20 °C to 100 °C. Preferably, the solution in the reactor may be stirred at 50 °C to 90 °C. More preferably, the solution the reactor may be stirred at 70 °C to 90 °C.

According to one embodiment, (iv) stirring the solution in the reactor (S4200) may be performed for approximately 1, 2, 3, 4, 5, 6, 7, 8, 9, 10, 12, 14, 16, 18, 20, 22, 24, 28, 32, 36, 40, 45, or 50 hours, or for longer than these times values. In a specific embodiment, (iv) stirring the solution in the reactor (S4200) may be performed for approximately 6, 7, 8, 9, 10, 12, 14, 16, 18, 20, 22, or 24 hours, or for longer than these times values.

According to one embodiment, materials that can control the pH of the solution may be further added such that the pH of the solution in the reactor becomes the above-described pH value.

### (v) Steps that can be selectively included

Hereinafter, steps that can be selectively included in the method for preparing whitlockite crystals according to the third embodiment will be described. For example, the method for preparing whitlockite crystals according to the third embodiment may selectively further include washing the resulting product, drying the resulting product, and grinding the resulting product. Here, the washing may be performed to remove ions remaining in the solution after the reaction is completed, but the present invention is not limited thereto.

In one embodiment, the method for preparing whitlockite crystals according to the third embodiment may further include washing the resulting product obtained in (iv).

In one embodiment, the method for preparing whitlockite crystals according to the third embodiment may further include drying the resulting product obtained in (iv).

In one embodiment, the method for preparing whitlockite crystals according to the second embodiment may further include (v) washing the resulting product obtained in (iv); and (vi) drying the resulting product obtained in (v).

In one embodiment, the washing may be performed by a method of filtering solids in the resulting product, but the present invention is not limited thereto. For example, the washing may be performed by a method of filtering solids in the resulting product using a filter press and removing a liquid in the resulting product or ions remaining in the liquid.

Through the method according to the present embodiment (third embodiment), whitlockite may be prepared. When crystals prepared by the method according to this embodiment are analyzed by XRD, more than 90 wt% of the prepared crystals may be identified as whitlockite crystals. In one embodiment, the XRD analysis results of the crystals prepared by the method according to this embodiment may show that 90, 91, 92, 93, 94, 95, 96, 97, 98, 99, or 99.9 wt% or more of the prepared crystals are whitlockite crystals. In a specific example, the XRD analysis results of the crystals prepared by the method according to this embodiment may show that 99 wt% or 99.9 wt% or more of the prepared crystals are whitlockite crystals. In a specific embodiment, the XRD analysis results of the crystals prepared by the method according to this embodiment may show that 100 wt% of the prepared crystals are whitlockite crystals.

In one embodiment, 90, 91, 92, 93, 94, 95, 96, 97, 98, 99, or 99.9 wt% or more of the solids produced by the method according to this embodiment may be whitlockite crystals. In a specific embodiment, 99 or 99.9 wt% or more of the solids produced by the method according to this embodiment may be whitlockite crystals. In a specific embodiment, all of the solids produced according to this embodiment may be whitlockite crystals.

### Fourth Embodiment

In the present application,
a method for preparing whitlockite crystals without the formation of hydroxyapatite crystals is provided, the method including:
(i) preparing a first solution containing phosphate ions in a reactor (S1300);
(ii) preparing a second solution containing calcium ions and magnesium ions (S2300);
(iii) adding the second solution to the reactor containing the first solution (S3300); and
(iv) stirring the solution in the reactor (S4300).

### (i) Preparing first solution containing phosphate ions in reactor (S1300)

The method according to one embodiment of the present application includes `preparing a first solution containing phosphate ions in a reactor (S1300).'

The first solution may be prepared by adding a phosphate ion feed material to a predetermined volume of solution. The phosphate ion feed material added to a predetermined volume of solution is ionized into phosphate ions and ions other than phosphate ions. In one embodiment, the first solution may include a first concentration of phosphate ions. For example, the first solution containing the first concentration of phosphate ions may be prepared by adding a first amount of phosphoric acid to a predetermined volume of solution.

The degree to which the phosphate ion feed material is ionized into phosphate ions in the first solution is changed by the amount of phosphate ion feed material, the degree of ionization of the phosphate ion feed material, the type of compound or ion contained in the first solution, the amount of each compound or ions, and/or the temperature of the solution. Alternatively, the degree to which the phosphate ion feed material is ionized into phosphate ions in the mixed solution to be described below is changed by the amount of phosphate ion feed material, the degree of ionization of the phosphate ion feed material, the type of compound or ion contained in the first solution, the amount of each compound or ion, and/or the temperature of the solution.

Here, the first solution containing phosphate ions is an acidic solution. That is, the pH of the first solution is 5 or less. According to one embodiment, the pH of the first solution may be approximately 5, 4.5, 4, 3.5, 3, 2.5, 2, 1.5, or 1 or less.

Here, according to one embodiment, the temperature of the first solution may be approximately 10, 15, 20, 30, 35, 40, 45, 50, 55, 60, 65, 70, 75, 80, 85, 90, 95, or 100 °C, but there is no limitation as long as the temperature can achieve the purpose of the present application.

### (ii) Preparing second solution containing calcium ions and magnesium ions(S2300)

Subsequently, the method includes `preparing a second solution containing calcium ions and magnesium ions (S2300).'

The second solution may be prepared by adding a predetermined amount of calcium ion feed material and a predetermined amount of magnesium ion feed material to a predetermined volume of solution. The added calcium ion feed material and magnesium ion feed material are ionized into calcium ions, magnesium ions, and other ions. In one embodiment, the second solution may include a second concentration of calcium ions and a third concentration of magnesium ions. For example, the second solution containing the second concentration of calcium ions and the third concentration of magnesium ions may be prepared by adding a second amount of calcium hydroxide and a third amount of magnesium hydroxide to a predetermined volume of solution.

In the second solution, the degrees to which the calcium ion feed material and the magnesium ion feed material are ionized into calcium ions and magnesium ions are changed by the amount of calcium ion feed material and the amount of magnesium ion feed material, the ionization degrees of the calcium ion material and the magnesium ion feed material, the type of compound or ion contained in the second solution, the amount of each compound or ion, and/or the temperature of the solution. Alternatively, in a mixed solution to be described below, the degrees to which calcium ion feed material and a magnesium ion feed material are ionized into calcium ions and magnesium ions is changed by the amount of calcium ion feed material and the amount of magnesium ion feed material, the ionization degrees of the calcium ion material and the magnesium ion feed material, the type of compound or ion contained in the mixed solution, the amount of each compound or ion, and/or the temperature of the solution.

Here, the second solution containing calcium ions and magnesium ions is a basic solution. That is, the pH of the second solution is more than 7. According to one embodiment, the pH of the second solution may be 8, 8.5, 9, 9.5, 10, 10.5, 11, 11.5, 12, 12.5, or 13 or more.

Here, according to one embodiment, the temperature of the second solution may be approximately 10, 15, 20, 25, 30, 35, 40, 45, 50, 55, 60, 65, 70, 75, 80, 85, 90, 95, or 100 °C, but there is no limitation as long as the temperature can achieve the purpose of the present application.

### (iii) Adding second solution to the reactor containing the first solution (S3300)

As described above, to prevent the formation of hydroxyapatite crystals by maintaining the pH of the solution in the reactor at 5 or less, a basic solution should be added to the reactor containing the acidic solution.

Accordingly, the method according to one embodiment of the present application includes `adding a second solution to the reactor containing the first solution (S3300).'

A mixed solution in which the first solution and the second solution are mixed is prepared by adding the second solution to the reactor containing the first solution.

Whitlockite is composed of phosphorous atoms (P), calcium atoms (Ca), magnesium atoms (Mg), and other atoms (hydrogen and oxygen atoms). To synthesize high-purity whitlockite, a specific ratio between phosphorous atoms, calcium atoms, and magnesium atoms used in this method may be required.

In one embodiment, the mixed solution in which the first solution and the second solution are mixed may include a first amount of phosphorous atoms, a second amount of calcium atoms, and a third amount of magnesium atoms. The ratio (P/Ca) of the amount of phosphorus atoms and the amount of calcium atoms, which can be included in the mixed solution, the ratio (Ca/Mg) of the amount of calcium atoms and the amount of magnesium atoms, which can be included in the mixed solution, the ratio (P/Mg) of the amount of phosphorus atoms and the amount of magnesium atoms, which can be included in the mixed solution, and the ratio (P/Ca+Mg) of the amount of phosphorous atoms and the amount of magnesium atoms+calcium atoms, which can be included in the mixed solution, will be described in detail in related paragraphs below.

In one embodiment, the first solution may include a first concentration of phosphate ions, and the second solution may include a second concentration of calcium ions and a third concentration of magnesium ions.

As described above, to achieve the purpose of the present invention, the pH of the solution in the reactor should be maintained at 5 or less. That is, the pH of the mixed solution in which the first and second solutions are mixed should be maintained at 5 or less.

Here, the concentration of phosphate ions included in the first solution and the concentration of calcium ions and the concentration of magnesium ions included in the second solution affect the pH of the mixed solution. Accordingly, the relationship between the first concentration, the second concentration, and the third concentration should be determined such that the pH of the mixed solution is 5 or less, and appropriate values of the first, second, and third concentrations should be selected based on the determined relationship.

Since the degree to which the phosphate ion feed material added to prepare the first solution in (i) is ionized in the mixed solution, and the degrees to which the calcium ion feed material and the magnesium ion feed material added to prepare the second solution in (ii) are ionized in the mixed solution may affect the pH of the mixed solution, the relationship between the first concentration, the second concentration, and the third concentration may be determined such that the pH of the mixed solution is 5 or less by considering the degree to which each ion feed material is ionized in the mixed solution.

The volume of the first solution and the volume of the second solution may also affect the pH of the mixed solution. Accordingly, by considering the volumes of the first and second solutions, the relationship between the first concentration, the second concentration, and the third concentration may be determined such that the pH of the mixed solution is 5 or less.

As described above, since the purpose of the present application is to prevent hydroxyapatite crystals from being formed in the preparation of whitlockite crystals, the case in which the pH of a localized portion of the solution in the reactor rises above 5 while adding the second solution to the first solution or the case in which the pH in the solution rises above 5 and then decreases to 5 or less before a certain period of time has elapsed also falls within the scope of the present application.

According to one embodiment, the addition of the second solution to the reactor containing the first solution starts at a first time point, and the pH of the solution in the reactor immediately after a second period of time has elapsed from the first time point may be 5.5, 5, 4.5, 4, 3.5, 3, 2.5, or 2 or less. Preferably, the pH of the solution in the reactor immediately after the second period of time has elapsed from the first time point is 4.5, 4, 3.5, 3, 2.5, or 2 or less.

The volumes of the first and second solutions are not particularly limited. For example, the volume of the first solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. For example, the volume of the second solution may be approximately 1 mL, 10 mL, 20 mL, 50 mL, 100 mL, 200 mL, 500 mL, 1 L, 2 L, 5 L, 10 L, 100 L, or 1000 L or more, but the present invention is not particularly limited thereto. In one embodiment, the volume ratio of the first and second solutions (the volume of the first solution /the volume of the second solution) may be approximately 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.2, 3.4, 3.6, 3.8, 4, 4.5, 5, 5.5, 6, 6.5, 7, 7.5, 8, 9, 10, 20, 30, 40, 50, or 100, but the present invention is not particularly limited thereto.

According to one embodiment, the second solution may be added to the reactor containing the first solution by pouring. According to one embodiment, the second solution may be added to the reactor containing the first solution for a certain period of time. According to one embodiment, the second solution may be added to the reactor containing the first solution at a certain rate for a certain period of time. The second solution may be added by a method usually used in the art.

According to the method disclosed in the conventional literature, since the solution in the reactor has to maintain basic conditions for a certain period of time or more, an acidic solution may be added while suppressing the addition rate, or added dropwise. As disclosed in the conventional literature, the addition of the acidic solution is performed for 40 minutes.

On the other hand, according to the method of preparing whitlockite crystals according to one embodiment of the present application, it is not necessary to reduce the addition rate of the basic second solution. Accordingly, by the method disclosed in the present application, the time required to prepare whitlockite crystals is reduced by shortening the addition time of the solution.

According to one embodiment of the present application, the second solution may be added to the reactor containing the first solution for a first period of time. According to one embodiment, the first period of time may be approximately 1200, 1100, 1000, 900, 800, 700, 600, 500, 400, 350, 300, 260, 220, 180, 160, 140, 120, 100, 80, 60, 50, 40, 30, 20, or 10 seconds. According to one embodiment, the first period of time may be 1200, 1100, 1000, 900, 800, 700, 600, 500, 400, 350, 300, 260, 220, 180, 160, 140, 120, 100, 80, 60, 50, 40, 30, 20, or 10 seconds or less. In a specific embodiment, the first period of time may be approximately 180, 160, 140, 120, 100, 80, 60, 50, 40, 30, 20, or 10 seconds. In a specific embodiment, the first period of time may be 180, 160, 140, 120, 100, 80, 60, 50, 40, 30, 20, or 10 seconds or less.

Here, according to one embodiment, during the process of adding the second solution, the solution in the reactor may be stirred. During the addition of the second solution, the materials included in the solution in the reactor may be properly mixed through stirring.

### (iv) Stirring the solution in the reactor (S4100)

The method for preparing whitlockite crystals according to one embodiment of the present application includes `(iv) stirring the solution in the reactor (S4100).' This process may include aging the solution to prepare whitlockite crystals.

As described above, the solution in the reactor should be maintained under acidic conditions. According to one embodiment, the pH of the solution in the reactor may be 5.5, 5, 4.5, 4, 3.5, 3, 2.5, or 2 or less. Preferably, the pH of the solution in the reactor is 4.5, 4, 3.5, 3, or 2.5 or less.

As described above, when whitlockite crystals are synthesized by the method disclosed in the previous art, hydroxyapatite crystals need to be produced, so the solution in the reactor has to maintain basic conditions for a certain period of time or more while containing some of the raw materials required for preparing whitlockite crystals. Accordingly, in the method disclosed in the previous art, an acidic phosphoric acid solution is added to the basic solution, and the addition rate of the acidic phosphoric acid solution is suppressed such that the solution in the reactor maintains basic conditions for a certain period of time or more.

On the other hand, according to the method provided by the present application, whitlockite crystals are prepared without the formation of hydroxyapatite crystals in the preparation of whitlockite crystals. Accordingly, by the method provided by the present application, the solution in the reactor does not need to maintain basic conditions for a certain period of time or more while containing the raw materials.

Since the purpose of the present application is to prevent the formation of hydroxyapatite crystals, according to one embodiment, after adding the second solution, the described purpose may be achieved by preventing the pH of the solution in the reactor from being maintained more than 5 for a certain period of time or more.

According to one embodiment of the present application, the addition of the second solution is terminated at a second time point, and the pH of the solution in the reactor immediately after a third period of time has elapsed from the second time point may be 5.5, 5, 4.5, 4, 3.5, 3, 2.5, or 2 or less. Preferably, the pH of the solution in the reactor immediately after a third period of time has elapsed from the second time point may be 4.5, 4, 3.5, 3, 2.5, or 2 or less.

To prepare whitlockite crystals, after terminating the addition of the second solution, the materials contained in the solution in the reactor may be properly mixed through stirring.

According to one embodiment, here, the solution in the reactor may be stirred at approximately 30, 35, 40, 45, 50, 55, 60, 65, 70, 75, 80, 85, 90, 95, or 100 °C. According to one embodiment, the solution in the reactor may be stirred at 20 °C to 100 °C. Preferably, the solution in the reactor may be stirred at 50 °C to 90 °C. More preferably, the solution in the reactor may be stirred at 70 °C to 90 °C.

According to one embodiment, (iv) stirring the solution in the reactor (S4100) may be performed for approximately 1, 2, 3, 4, 5, 6, 7, 8, 9, 10, 12, 14, 16, 18, 20, 22, 24, 28, 32, 36, 40, 45, or 50 hours, or for longer than these times values. In a specific embodiment, (iv) stirring the solution in the reactor (S4100) may be performed for approximately 6, 7, 8, 9, 10, 12, 14, 16, 18, 20, 22, or 24 hours, or for longer than these times values.

According to one embodiment, materials that can control the pH of the solution may be further added such that the pH of the solution in the reactor becomes the above-described pH value.

### (v) Steps that can be optionally included

Hereinafter, steps that can be optionally included in the method for preparing whitlockite crystals according to the fourth embodiment will be described. For example, the method for preparing whitlockite crystals according to the fourth embodiment may selectively further include washing the resulting product, drying the resulting product, and grinding the resulting product. Here, the washing may be performed to remove ions remaining in the solution after the reaction is completed, but the present invention is not limited thereto.

In one embodiment, the method for preparing whitlockite crystals according to the fourth embodiment may further include washing the resulting product obtained in (iv).

In one embodiment, the method for preparing whitlockite crystals according to the fourth embodiment may further include drying the resulting product obtained in (iv).

In one embodiment, the method for preparing whitlockite crystals according to the fourth embodiment may further include (v) washing the resulting product obtained in (iv); and (vi) drying the resulting product obtained in (v).

In one embodiment, the washing may be performed by a method of filtering solids in the resulting product, but the present invention is not limited thereto. For example, the washing may be performed by a method of filtering solids in the resulting product using a filter press and removing a liquid in the resulting product or ions remaining in the liquid.

Through the method according to the present embodiment (fourth embodiment), whitlockite may be prepared. When crystals prepared by the method according to this embodiment are analyzed by XRD, more than 90 wt% of the prepared crystals may be identified as whitlockite crystals. In one embodiment, the XRD analysis results of the crystals prepared by the method according to this embodiment may show that 90, 91, 92, 93, 94, 95, 96, 97, 98, 99, or 99.9 wt% or more of the prepared crystals are whitlockite crystals. In a specific example, the XRD analysis results of the crystals prepared by the method according to this embodiment may show that 99 wt% or 99.9 wt% or more of the prepared crystals are whitlockite crystals. In a specific embodiment, the XRD analysis results of the crystals prepared by the method according to this embodiment may show that 100 wt% of the prepared crystals are whitlockite crystals.

In one embodiment, 90, 91, 92, 93, 94, 95, 96, 97, 98, 99, or 99.9 wt% or more of the solids produced by the method according to this embodiment may be whitlockite crystals. In a specific embodiment, 99 or 99.9 wt% or more of the solids produced by the method according to this embodiment may be whitlockite crystals. In a specific embodiment, all of the solids produced by the method according to this embodiment may be whitlockite crystals.

### Ratio of phosphorus (P) atoms, calcium (Ca) atoms, and magnesium (Mg) atoms used in the method for preparing whitlockite crystals provided by the present application

The ratio of an amount of phosphorus (P) atoms, an amount of calcium (Ca) atoms, and an amount of magnesium (Mg) atoms used in the method for preparing whitlockite crystals may be determined by considering the following:
the ratio (P:Ca:Mg) of phosphorus atoms, calcium atoms, and magnesium atoms constituting whitlockite is 14:18:2;
the pH of the mixed solution should be maintained as 5 or less;
more than 90 wt% (e.g., 91, 92, 93, 94, 95, 96, 97, 98, 99, or 99.9 wt% or more) of the solids produced as the result of the reaction are whitlockite crystals; and/or
whether or not the time required to prepare whitlockite crystals can be shortened.

For example, in one embodiment, more P than the appropriate amount of P determined based on the ratio of the numbers of P/Ca/Mg atoms included in whitlockite may be used in the method for preparing whitlockite crystals provided by the present application. This is because more phosphoric acid may be used to ensure the pH in the reactor where whitlockite crystals are prepared can be maintained at 5 or less throughout the entire process. As a specific example, the ratio (P/Ca) of phosphorous atoms to calcium atoms included in whitlockite may be approximately 0.78, however the ratio (P/Ca) of the total amount of phosphorus atoms to the total amount of calcium atoms used to prepare whitlockite crystals may be more than 0.78. In another specific example, the ratio (P/Ca+Mg) of phosphorus atoms to calcium atoms+magnesium atoms included in whitlockite may be approximately 0.7, or the ratio (P/Ca+Mg) of phosphorus atoms to calcium atoms+magnesium atoms used to prepare whitlockite crystals may be more than 0.7.

Hereinafter, embodiments of the ratios between phosphorus atoms, calcium atoms, and magnesium atoms used to prepare whitlockite crystals are disclosed. Here, the atoms used to prepare whitlockite crystals include not only atoms that form whitlockite crystals after whitlockite synthesis is completed but also atoms that are present in the form of ions in the solution.

As described above, to synthesize pure whitlockite through the method of the present application, an appropriate ratio between phosphorous atoms, calcium ions, and magnesium ions may be required.

Here, the amount of phosphorus atoms used herein may mean the number of phosphorus atoms included in the entire phosphate ion feed material used to prepare whitlockite crystals, but the present invention is not particularly limited thereto. For example, the amount of phosphorus atoms used herein may be a value obtained by converting the number of phosphorus atoms included in the entire phosphate ion feed material used to prepare whitlockite crystals to the number of moles.

The amount of calcium atoms used herein may refer to the number of calcium atoms included in the entire calcium ion feed material used to prepare whitlockite crystals, but the present invention is not particularly limited thereto. For example, the amount of calcium atoms used herein may be a value obtained by converting the number of calcium atoms included in the entire calcium ion feed material used to prepare whitlockite crystals to the number of moles.

The amount of magnesium atoms used herein may refer to the number of magnesium atoms included in the entire magnesium ion feed material used to prepare whitlockite crystals, but the present invention is not particularly limited thereto. For example, the amount of magnesium ions used herein may be a value obtained by converting the number of magnesium atoms included in the entire magnesium ion feed material used to prepare whitlockite crystals to the number of moles.

In one embodiment, the ratio (P/Ca) of the amount of phosphorus atoms to the amount of calcium atoms used to prepare whitlockite crystals according to the method of the present application may be 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.5, 4, 5, 6, 7, 8, 9, or 10 or more. In one embodiment, the ratio (P/Ca) of the amount of phosphorus atoms to the amount of calcium atoms used herein may be approximately 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.2, 3.4, 3.6, 3.8, 4, 4.5, 5, 6, 7, 8, 9, or 10 or in a range set by two values selected from aforesaid values. In one embodiment, the ratio (P/Ca) of the amount of phosphorous atoms and the amount of calcium ions used herein may be 0.5 to 1, 1 to 2, 2 to 3, 3 to 4, 4 to 5, 5 to 6, 6 to 7, 7 to 8, 8 to 9, or 9 to 10. In a specific embodiment, the ratio (P/Ca) of the amount of phosphorus atoms to the amount of calcium atoms used herein may be 1, 1.1, 1.2, 1.3, 1.4, or 1.5 or more. In a specific embodiment, the ratio (P/Ca) of the amount of phosphorus atoms to the amount of calcium atoms used herein may be 1 to 1.8, 1.2 to 1.6, or 1.3 to 1.5. In a specific embodiment, the ratio (P/Ca) of phosphorus atoms to calcium atoms used herein may be approximately 1.3, 1.4, or 1.5.

Here, as described above, the ratio of phosphorus atoms to calcium atoms used herein may be calculated from, for example, the ratio of the number of phosphorous atoms included in the phosphate ion feed material used to prepare whitlockite to the number of calcium ions included in the calcium ion feed material used to prepare whitlockite.

In another example, the ratio of the amount of phosphorous atoms to the amount of calcium atoms used herein may be calculated from the ratio of the amount of phosphorous atoms included in a mixed solution (i.e., a solution prepared after mixing both the acidic solution and the basic solution) to the amount of calcium atoms included in the mixed solution. Here, the amount of phosphorus atoms included in the mixed solution may be calculated by considering both phosphorus atoms constituting ions such as phosphate ions and phosphorus atoms constituting solid crystals with other atoms. For example, when there are phosphate ions (PO₄⁻, this case may be calculated as one atom per one phosphate ion) and whitlockite crystals in the mixed solution, the amount of phosphorus atoms included in the mixed solution may be calculated by considering both the amount of phosphorous atoms constituting phosphate ions and the amount of phosphorus atoms constituting whitlockite crystals. Likewise, here, the amount of calcium atoms included in the mixed solution may be calculated by considering both calcium atoms constituting ions such as calcium ions and calcium atoms constituting solid crystals with other atoms.

In one embodiment, the ratio (Ca/Mg) of calcium atoms to magnesium atoms used to prepare whitlockite crystals according to the method of the present application may be 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.2, 2.4, 2.6, 2.8, 3, 3.5, 4, or 5 or more. In one embodiment, the ratio (Ca/Mg) of calcium atoms to magnesium atoms used to prepare whitlockite crystals according to the method of the present application may be approximately 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.2, 3.4, 3.6, 3.8, 4, 4.5, or 5 or in a range set by two values selected from aforesaid values. In one embodiment, the ratio (Ca/Mg) of calcium atoms to magnesium atoms used to prepare whitlockite crystals according to the method of the present application may be 0.5 to 1, 1 to 2, 2 to 3, 3 to 4, or 4 to 5. In a specific embodiment, the ratio (Ca/Mg) of calcium atoms to magnesium atoms used to prepare whitlockite crystals according to the method of the present application may be 2.3, or 2.5 or more. In a specific embodiment, the ratio (Ca/Mg) of calcium atoms to magnesium atoms used to prepare whitlockite crystals according to the method of the present application may be 2.2 to 4.0, 2.2 to 3.5, 2.5 to 3.5, or 2.5 to 3.0.

Here, as described above, the ratio of the amount of calcium atoms to the amount of magnesium atoms used herein may be calculated from, for example, the ratio of the number of calcium atoms included in the calcium ion feed material used to prepare whitlockite to the number of magnesium atoms included in the magnesium ion feed material used to prepare whitlockite.

In another example, the ratio of the amount of calcium atoms to the amount of magnesium atoms used herein may be calculated from the ratio of the amount of calcium atoms included in a mixed solution (i.e., a solution prepared after mixing both the acidic solution and the basic solution) to the amount of magnesium atoms included in the mixed solution. Here, the amount of calcium atoms included in the mixed solution may be calculated by considering both calcium atoms constituting solid crystals with other atoms and calcium atoms constituting Ca²⁺ ions (in this case, one calcium atom per one Ca²⁺). For example, when there are Ca²⁺ ions and whitlockite crystals in the mixed solution, the amount of calcium atoms included in the mixed solution may be calculated by considering both the amount of calcium atoms constituting Ca²⁺ ions and the amount of calcium atoms constituting whitlockite crystals. Likewise, here, the amount of magnesium atoms included in the mixed solution may be calculated by considering both magnesium atoms constituting solid crystals with other atoms and magnesium atoms constituting Mg²⁺ ions.

In one embodiment, the ratio (P/Mg) of phosphorous atoms to magnesium atoms, used to prepare whitlockite crystals according to the method of the present application, may be 1, 1.1, 1.2, 1.3, 1.4, 1.5, 1.6, 1.7, 1.8, 1.9, 2, 2.2, 2.4, 2.6, 2.8, 3, 3.5, 4, 5, 6, 7, 8, 9, 10, 12, 14, 16, or 20 or more. In one embodiment, the ratio (P/Mg) of phosphorous atoms to magnesium atoms, used to prepare whitlockite crystals according to the method of the present application may be approximately 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.8, 6, 6.5, 7, 7.5, 8, 8.5, 9, 9.5, or 10 or in a range set by two values selected from aforesaid values. In one embodiment, the ratio (P/Mg) of phosphorous atoms to magnesium atoms, used to prepare whitlockite crystals according to the method of the present application may be 1 to 2, 2 to 3, 3 to 4, 4 to 5, 5 to 6, 6 to 7, 7 to 8, 8 to 9, or 9 to 10. In a specific embodiment, the ratio (P/Mg) of phosphorus atoms to magnesium atoms used to prepare whitlockite crystals according to the method of the present application may be 3.2, 3.4, 3.6, 3.8, 4.0, 4.2, or 4.4 or more. In a specific embodiment, the ratio (P/Mg) of phosphorous atoms to magnesium atoms, used to prepare whitlockite crystals according to the method of the present application may be 3.0 to 5.5, 3.1 to 5.4, 3.3 to 5, or 3.5 to 4.0.

Here, as described above, the ratio of the amount of phosphorus atoms to the amount of magnesium atoms used herein may be calculated from, for example, the ratio of the number of phosphorus atoms included in the phosphate ion feed material used to prepare whitlockite to the number of magnesium atoms included in the magnesium ion feed material used to prepare whitlockite.

In another example, the ratio of the amount of phosphorus atoms to the amount of magnesium atoms used herein may be calculated from the ratio of the amount of phosphorus atoms contained in a mixed solution (i.e., a solution prepared after mixing both the acidic solution and the basic solution) to the amount of magnesium atoms contained in the mixed solution. For example, the amount of magnesium atoms contained in the mixed solution may be calculated by considering both magnesium atoms constituting solid crystals with other atoms and magnesium atoms constituting Mg²⁺ ions (in this case, one magnesium atom per one Mg²⁺). For example, when there are Mg²⁺ ions and whitlockite crystals in the mixed solution, the amount of magnesium atoms contained in the mixed solution may be calculated by considering both the amount of magnesium atoms constituting Mg²⁺ ions and the amount of magnesium atoms constituting whitlockite crystals. Likewise, here, the amount of phosphorus atoms contained in the mixed solution may be calculated by considering both phosphorus atoms constituting solid crystals with other atoms and phosphorus atoms constituting phosphate ions.

In one embodiment, the ratio (P/Ca+Mg) of phosphorus atoms to cation atoms used to prepare whitlockite crystals according to the method of the present application may be 0.5, 0.6, 0.7, 0.8, 0.9, 1, 1.1, 1.2, 1.3, 1.4, 1.5, 1.6, 1.7, 1.8, 1.9, 2.0, 2.2, 2.4, 2.6, 2.8, 3.0, 3.2, 3.4, 3.6, 3.8, 4.0, 4.2, 4.4, 4.6, 4.8, or 5 or more. In one embodiment, the ratio (P/Ca+Mg) of phosphorus atoms to cation atoms used to prepare whitlockite crystals according to the method of the present application may be approximately 0.5, 0.6, 0.7, 0.8, 0.9, 1, 1.1, 1.2, 1.3, 1.4, 1.5, 1.6, 1.7, 1.8, 1.9, 2.0, 2.2, 2.4, 2.6, 2.8, 3.0, 3.2, 3.4, 3.6, 3.8, 4.0, 4.2, 4.4, 4.6, 4.8, or 5 or in a range set by two values selected from aforesaid values. In one embodiment, the ratio (P/Ca+Mg) of phosphorus atoms to cation atoms used to prepare whitlockite crystals according to the method of the present application may be 0.4 to 0.8, 0.8 to 1.2, 1.2 to 1.6, or 1.6 to 2.0, but the present invention is not limited thereto. In a specific embodiment, the ratio (P/Ca+Mg) of phosphorus atoms to cation atoms used to prepare whitlockite crystals according to the method of the present application may be approximately 0.95, 0.96, 0.97, 0.98, 0.99, 1.0, 1.01, 1.02, 1.03, 1.04, 1.05, 1.06, 1.07, 1.08, 1.09, or 1.1 or in a range set by two values selected from aforesaid values. In a specific embodiment, the ratio (P/Ca+Mg) of phosphorus atoms to cation atoms used to prepare whitlockite crystals according to the method of the present application may be 0.94 to 1.1, 0.95 to 1.09, or 0.98 to 1.02.

### Whitlockite crystals prepared by method for preparing whitlockite crystals provided by the present application

According to the method provided by the present application, hydroxyapatite crystals are not produced during the preparation of whitlockite crystals. Accordingly, when whitlockite crystals are prepared by the method provided by the present application, the prepared whitlockite crystals are not transformed from hydroxyapatite crystals.

According to one embodiment of the present application, the whitlockite crystals may be formed by (a) direct precipitation from a solution in a reactor and/or (b) transformation from crystals containing calcium atoms and/or magnesium atoms other than hydroxyapatite crystals.

According to one embodiment of the present application, the whitlockite crystals may be formed by any one selected from (a), (b), and (c) below:
(a) direct precipitation from a solution in a reactor;
(b) transformation from crystals containing calcium atoms and/or magnesium atoms, other than hydroxyapatite crystals; or
(c) both (a) and (b).

According to one embodiment, the crystals containing calcium atoms and/or magnesium atoms, other than hydroxyapatite crystals, may include dihydrate calcium phosphate crystals, calcium phosphate crystals, calcium hydroxide crystals, magnesium phosphate crystals, and magnesium hydroxide crystals, but the present invention is not limited thereto.

According to one embodiment, the crystals containing calcium atoms and/or magnesium atoms, other than hydroxyapatite crystals, may include β-tricalcium phosphate (Ca₃(PO₄)₂), monetite (Ca(HPO₄)), brushite (Ca(HPO₄)(H2O)₂), portlandite (Ca(OH)₂), newberyite (MgHPO₄(H₂O)₃), and brucite (Mg(OH)₂), but the present invention is not limited thereto.

According to one embodiment of the present application, the average diameter of the whitlockite crystals prepared by the method provided by the present application may be 10 nm to 1000 nm. According to one embodiment, the average diameter of the prepared whitlockite crystals may be 20 nm to 500 nm. According to one embodiment, the average diameter of the prepared whitlockite crystals may be 30 nm to 200 nm. According to one embodiment, the average diameter of the prepared whitlockite crystals may be 30 nm to 100 nm. According to one embodiment, the average diameter of the prepared whitlockite crystals may be 30 nm to 60 nm. According to one embodiment, the average diameter of the prepared whitlockite crystals may be approximately 10, 15, 20, 25, 30, 35, 40, 45, 50, 55, 60, 65, 70, 75, 80, 85, 90, 95, 100, 110, 120, 140, 160, 200, 250, 300, 400, 500, 600, 700, 800, 900, or 1000 nm or in a range set by two values selected from aforesaid values (e.g., 10 nm to 1000 nm, 30 nm to 100 nm, or 20 nm to 60 nm). Preferably, the average diameter of the prepared whitlockite crystals may be approximately 40, 45, 50, 55, 60, 65, 70, 75, 80, or 85 nm or in a range set by two values selected from aforesaid values.

### Effects of method for preparing whitlockite crystals provided by the present application

Whitlockite crystals may be prepared faster using the method for preparing whitlockite crystals of the present application than when using the conventional method.

High-purity whitlockite crystals may be prepared faster using the method for preparing whitlockite crystals of the present application than when using the conventional method. Here, the high-purity whitlockite crystals mean that crystals containing calcium atoms and/or magnesium atoms of a solution in a reactor include only whitlockite crystals.

According to the method disclosed in the previous art, the time required to prepare high-purity whitlockite crystals is at least 360 minutes or longer. On the other hand, when whitlockite is prepared by the method provided by the present application, the time required to prepare high-purity whitlockite crystals may be shortened for the following reasons.

According to the method provided by the present application, the time required to prepare high-purity whitlockite crystals may be further shortened by shortening the addition time of a cation feed material.

According to the method provided by the present application, the time required to prepare high-purity whitlockite crystals may be further shortened by shortening the addition time of a second solution.

According to the method provided by the present application, hydroxyapatite crystals are not produced during the preparation of whitlockite crystals, so the time required to prepare high-purity whitlockite crystals may be further shortened.

According to the method provided by the present application, whitlockite crystals are prepared under acidic conditions, so the time required to prepare high-purity whitlockite crystals may be further shortened.

According to one embodiment, high-purity whitlockite crystals may be prepared within a minimum of 30 minutes and a maximum of 300 minutes (5 hours) after the start of the addition of the cation feed material to the reactor containing the first solution. Preferably, high-purity whitlockite crystals may be prepared within a minimum of 30 minutes and a maximum of 180 minutes (3 hours) after the start of the addition of the cation feed material.

According to one embodiment of the present application, high-purity whitlockite crystals may be prepared within a minimum of 30 minutes and a maximum of 300 minutes (5 hours) after the start of the addition of the second solution to the reactor containing the first solution. Preferably, high-purity whitlockite crystals may be prepared within a minimum of 30 minutes and a maximum of 180 minutes (3 hours) after the start of the addition of the second solution.

### Experimental Examples

### Preparation Example. Preparation of whitlockite crystals

The inventors of the present application prepared whitlockite crystals according to Preparation Examples 1 to 4 without producing hydroxyapatite crystals.

### Preparation Example 1

After adding 4828.43 mL of deionized water to a first reactor (10 L), 171.57 mL of 85% phosphoric acid solution (Daejung Chemicals & Metals, Cat. No. 6532-4105 Phosphoric acid, 85%) was added. Subsequently, a phosphoric acid solution was prepared by increasing a temperature of the resulting solution to 80 °C for one hour while stirring at 250 rpm. Here, the pH of the phosphoric acid solution was measured at 1.3.

After adding 5000 mL of deionized water to a second reactor (10 L), 137.05 g of calcium hydroxide (Ca(OH)₂), 37.9 g of magnesium hydroxide (Mg(OH)₂) were added. Subsequently, a cationic aqueous solution was prepared by increasing a temperature of the resulting solution to 60 °C for one hour while stirring at 250 rpm. The pH of the cationic aqueous solution was measured while calcium hydroxide (Ca(OH)₂) and magnesium hydroxide (Mg(OH)₂) was not completely dissolved, and the measured pH was more than 12.

The cationic aqueous solution was added to the first reactor containing the phosphoric acid solution at 2.5 L/min. Here, the solution in the first reactor was stirred at 250 rpm.

After adding the cationic aqueous solution, the solution in the reactor was aged while stirring for 24 hours at 80 °C and 250 rpm. After the completion of the 24-hour aging, whitlockite crystals were washed to remove ions remaining in the solution. Here, a filter press method was used. After filter pressing, the resulting whitlockite cake was put into a dry oven and dried at 60 °C to 100 °C for 24 hours, thereby obtaining whitlockite crystals.

### Preparation Example 2

In Preparation Example 2, unlike Preparation Example 1, 41.69 g of magnesium hydroxide (Mg(OH)₂) was added.

The process for obtaining whitlockite crystals is the same as described in Preparation Example 1 except for the amount of magnesium hydroxide added.

### Preparation Example 3

In Preparation Example 3, unlike Preparation Example 1, 26.53 g of magnesium hydroxide (Mg(OH)₂) was added.

The process for obtaining whitlockite crystals is the same as described in Preparation Example 1 except for the amount of magnesium hydroxide added.

### Preparation Example 4

In Preparation Example 4, unlike Preparation Example 1, 49.27 g of magnesium hydroxide (Mg(OH)₂) was added.

The process for obtaining whitlockite crystals is the same as described in Preparation Example 1 except for the amount of magnesium hydroxide added.

### Experimental Example 1. Confirmation whether high-purity whitlockite crystals were produced in each Preparation Example

The inventors of the present application prepared whitlockite crystals according to Preparation Examples 1, 2, 3, and 4, and it was confirmed whether 100 wt% whitlockite (i.e., high-purity whitlockite) crystals were produced in each Preparation Example.

After obtaining whitlockite crystals according to Preparation Examples 1 to 4, the crystals obtained in each Preparation Example were analyzed using X-ray diffraction (XRD).

Conditions used for the analysis using XRD are as follows.
- Scan range 3' to 80'
- Scan rate 0.02
- Scan speed 3'/min
- 40 Kv
- 40 mA

As a result of analyzing the crystals obtained in Preparation Examples 1 to 4 through XRD, in the case of synthesis according to Preparation Examples 1 and 2, it can be seen that 100 wt% whitlockite crystals were obtained. That is, when whitlockite crystals were prepared according to Preparation Examples 1 and 2, high-purity whitlockite crystals were prepared.

The XRD analysis results showed that 100 wt% whitlockite crystals were obtained according to Preparation Example 1.

The XRD analysis results showed that 100 wt% whitlockite crystals were obtained according to Preparation Example 2.

The XRD analysis results showed that 50.3 wt% whitlockite crystals and 49.7 wt% calcium phosphate crystals were obtained according to Preparation Example 3.

The XRD analysis results showed that 85 wt% whitlockite crystals and 16 wt% magnesium phosphate crystals were obtained according to Preparation Example 4.

The conditions of each Preparation Example, the type and wt% of the prepared crystals are disclosed in Table 1 below.

**[Table 1] Conditions for Preparation Examples 1 to 4 and crystals prepared in each Preparation Example**

| | H₃PO₄ used | Ca(OH)₂ used | Mg(OH)₂ used | Addition rate | Prepared crystals |
|---|---|---|---|---|---|
| Preparation Example 1 | 171.57 mL | 137.05 g | 37.9 g | 2.5 L/min | WH 100 wt% |
| | (2.54 mol) | (1.85 mol) | (0.65 mol) | | |
| | (0.508M) | (0.37M) | (0.13M) | | |
| Preparation Example 2 | 171.57 mL | 137.05 g | 41.69 g | 2.5 L/min | WH 100 wt% |
| | (2.54 mol) | (1.85 mol) | (0.72 mol) | | |
| | (0.508M) | (0.37M) | (0.144M) | | |
| Preparation Example 3 | 171.57 mL | 137.05 g | 26.53 g | 2.5 L/min | WH 50.3 wt% |
| | (2.54 mol) | (1.85 mol) | (0.45 mol) | | CP 49.7 wt% |
| | (0.508M) | (0.37M) | (0.09M) | | |
| Preparation Example 4 | 171.57 mL | 137.05 g | 49.27 g | 2.5 L/min | WH 85 wt% |
| | (2.54 mol) | (1.85 mol) | (0.84 mol) | | MP 16 wt% |
| | (0.508M) | (0.37M) | (0.168M) | | |

In Table 1, WH denotes whitlockite crystals, HA denotes hydroxyapatite crystals, CP denotes calcium phosphate crystals, and MP denotes magnesium phosphate crystals. Further, each molar concentration (M) shown in Table 1 was calculated based on a phosphoric acid solution (5L) or a cationic aqueous solution (5L). Only when whitlockite crystals were prepared according to Preparation Examples 1 and 2, high-purity whitlockite crystals were prepared.

### Experimental Example 2. Change in pH of solution in reactor during preparation of whitlockite crystals according to Preparation Example 1

The inventors of the present application measured the change in pH of the solution in the reactor during the preparation of whitlockite crystals. The pH of the solution in the reactor was measured with a pH meter.

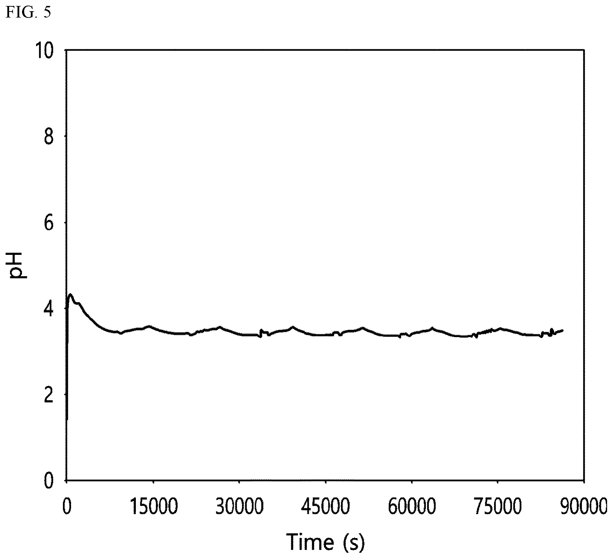

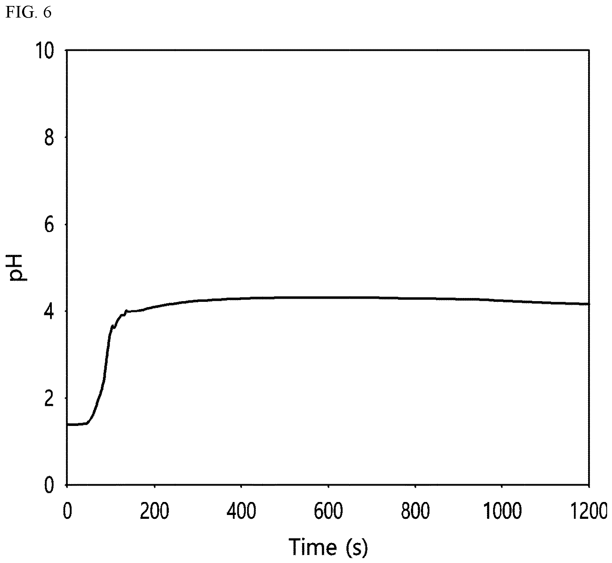

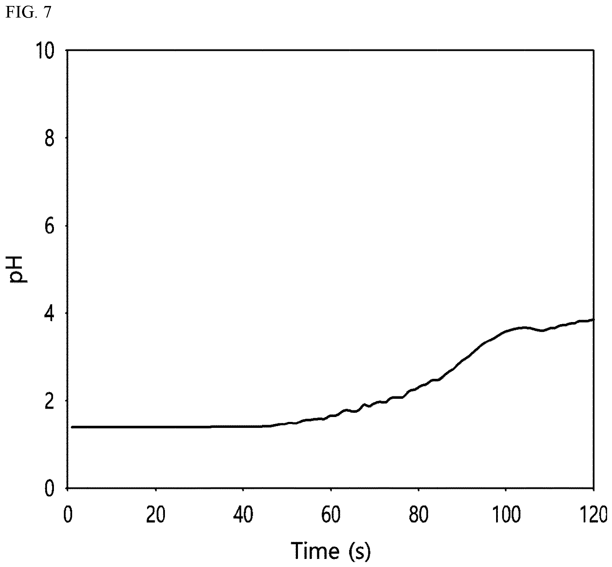

In the preparation of whitlockite crystals according to Preparation Example 1, the changes in pH of the solution in the reactor after the addition of a cationic aqueous solution began were measured. The result of measuring the pH changes is shown in FIG. 5. FIG. 6 is a graph showing only the result between 0 to 1200 seconds in the graph of FIG. 5. FIG. 7 is a graph showing only the result between 0 to 120 seconds in the graph of FIG. 5. In other words, FIGS. 5, 6, and 7 are the same graph showing the pH change in the reactor according to Preparation Example 1 except that the time scale on the x axis was changed to 90000 seconds, 1200 seconds, and 120 seconds, respectively.

As shown in FIGS. 5, 6, and 7, the pH of the solution in the reactor was maintained at 4.5 or less.

### Experimental Example 3. Whether hydroxyapatite crystals were produced during preparation of whitlockite crystals according to Preparation Example 1

The inventors of the present application observed whether hydroxyapatite crystals were produced during the preparation of whitlockite crystals.

In the preparation of whitlockite crystals according to Preparation Example 1, whether hydroxyapatite crystals were produced after the addition of a cationic aqueous solution was evaluated by observation.

At each time point, a portion of the solution during the reaction in the reactor was collected as a sample, and the sample collected at each time point was analyzed through X-ray diffraction (XRD). Here, the conditions used in XRD analysis are the same as those used in Experimental Example 1. Information on each sample is disclosed below.
- First sample: collected 54 seconds after the addition of the cationic aqueous solution began, in other words, it was collected at the time when 2.25 L of the cationic aqueous solution was added and obtained according to a predetermined process.
- Second sample: collected 67.2 seconds after the addition of the cationic aqueous solution began, in other words, it was collected at the time when 2.8 L of the cationic aqueous solution was added and obtained according to a predetermined process.
- Third sample: collected 91.2 seconds after the addition of the cationic aqueous solution began, in other words, it was collected at the time when 3.8 L of the cationic aqueous solution was added and obtained according to a predetermined process.
- Fourth sample: collected 110.2 seconds after the addition of the cationic aqueous solution began, in other words, it was collected at the time when 4.6 L of the cationic aqueous solution was added and obtained according to a predetermined process.
- Fifth sample: collected 20 minutes after the addition of the cationic aqueous solution and obtained according to a predetermined process.
- Sixth sample: collected 24 hours after the addition of the cationic aqueous solution began and obtained according to a predetermined process.

The first sample was obtained through the following process.
(1) collecting a predetermined volume of solution from the solution in the reactor 54 seconds after the addition of the cationic aqueous solution began, in other words, at the time when 2.26 L of the cationic aqueous solution was added.
(2) removing a supernatant after centrifuging the collected solution at 250G for 10 minutes using a centrifuge.
(3) after removing the supernatant, adding 99% ethanol and sufficiently mixing it, and removing a supernatant through centrifugation at 250G for 10 minutes.
(4) repeating the process of (3) three or four times and then drying the resulting product.
(5) obtaining the first sample.

The second to sixth samples were also obtained through a similar method to that for the first sample.

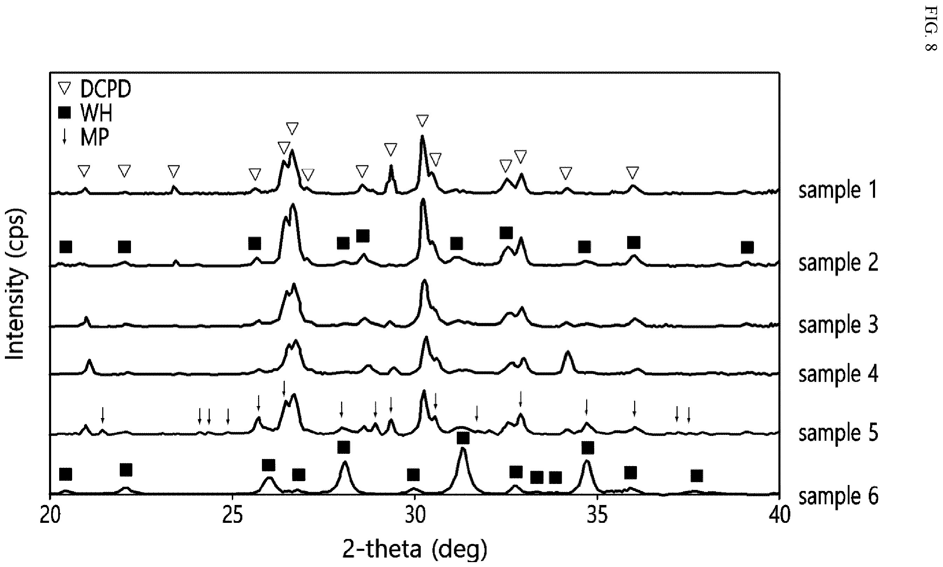

The result of analyzing each sample using XRD is disclosed in FIG. 8.

Referring to FIG. 8, it can be seen that no peaks indicating hydroxyapatite crystals are observed in the first, second, third, fourth, fifth, and sixth samples.

The results of analyzing the XRD graph are summarized in the following table.

**[Table 2] XRD graph analysis results**

| | **Sample 1** | **Sample 2** | **Sample 3** | **Sample 4** | **Sample 5** | **Sample 6** |
|---|---|---|---|---|---|---|
| Monetite Ca(HPO₄) | 90.6 wt% | 81.0 wt% | 79.3 wt% | 64.9 wt% | 44.6 wt% | - |
| Brushite Ca(OH)₂ | 9.4 wt% | 1.02 wt% | 3.7 wt% | 8.2 wt% | 4.6 wt% | - |
| Portlandite Ca(OH)₂ | - | - | - | 8.6 wt% | - | - |
| Newberyite Mg(PO₃OH)·3H₂O | - | - | - | - | 18.9 wt% | - |
| Hydroxyapatite Ca₁₀(PO₄)₅(OH)₂ | - | - | - | - | - | - |
| Whitlockite | - | 18.0 wt% | 17.0 wt% | 18.3 wt% | 32.0 wt% | 100 wt% |

As disclosed in FIG. 8 and Table 2, when whitlockite crystals are prepared according to Preparation Example 1, hydroxyapatite crystals were not observed during the preparation.

In Experimental Example 2, when whitlockite crystals are prepared according to Preparation Example 1, it was observed that the pH of the solution in the reactor was maintained at 4.5 or less even when a basic cationic aqueous solution was added. In addition, the XRD graph in FIG. 8 shows that, when whitlockite crystals are prepared according to Preparation Example 1, hydroxyapatite crystals are not produced.

That is, when whitlockite crystals are prepared under conditions in which a basic cationic aqueous solution is added to an acidic phosphoric acid solution and the solution in the reactor is maintained at pH of 5 or less, it can be seen that hydroxyapatite crystals are not produced during the preparation of whitlockite crystals.

### Experimental Example 4. Time required to prepare whitlockite crystals

In the preparation of whitlockite crystals according to Preparation Example 1, the inventors of the present application obtained samples from the reactor 3, 6, and 24 hours after addition of a cationic aqueous solution, analyzed the obtained samples using XRD, and photographed the obtained samples using a scanning electron microscope (SEM). Here, the conditions used for analysis through XRD are the same as those in Experimental Example 1.

At each time, the sample was obtained by collecting a predetermined volume of solution from the solution in the reactor and then drying it. The obtained sample was used for XRD analysis and SEM imaging.

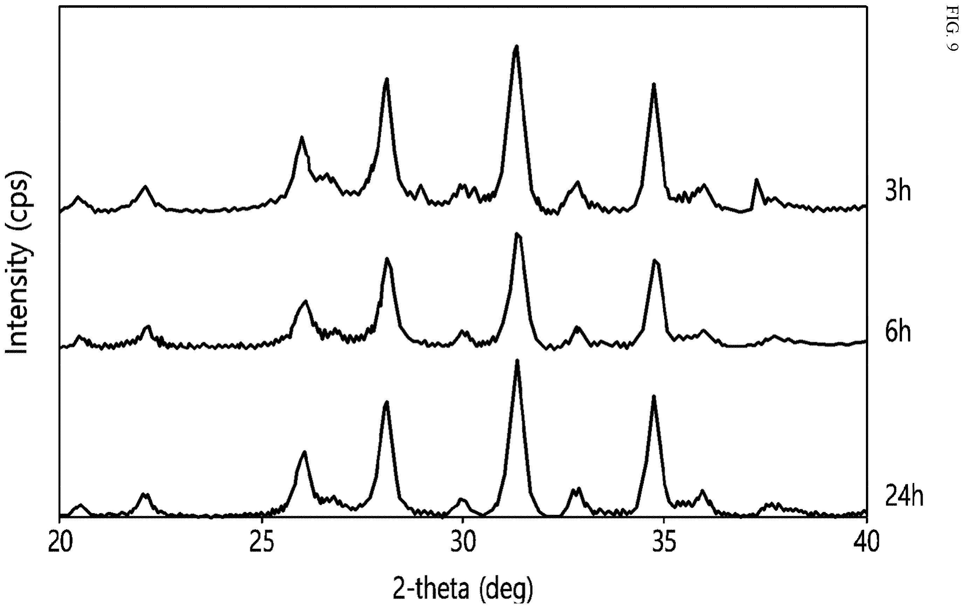

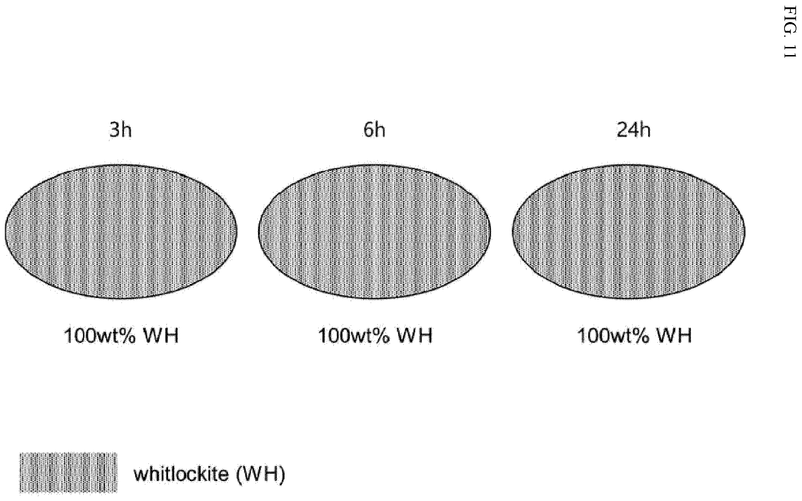

Referring to the XRD data in FIG. 9 and the XRD analysis results in FIG. 11, it can be seen that the samples obtained from the reactor at 3, 6, and 24 hours include 100% whitlockite crystals.

Accordingly, when whitlockite crystals are prepared according to Preparation Example 1, it can be seen that high-purity whitlockite crystals are synthesized within at least 3 hours. That is, when whitlockite crystals are prepared according to Preparation Example 1, 100% whitlockite crystals are synthesized within at least 3 hours after adding the cationic aqueous solution.

Therefore, when whitlockite crystals are prepared under acidic conditions without the production of hydroxyapatite crystals, it can be seen that high-purity whitlockite crystals are synthesized within at least 3 hours.

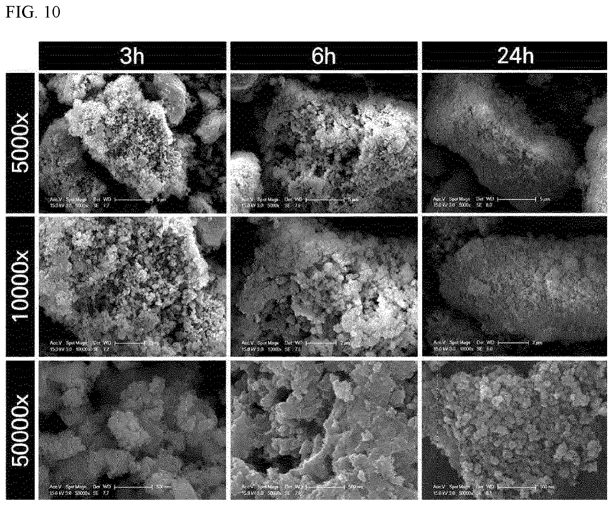

Whitlockite crystals having an average diameter of 72.36 nm were obtained from the sample collected 3 hours after addition of the cationic aqueous solution. Whitlockite crystals having an average diameter of 63.26 nm were obtained from the sample collected 6 hours after addition of the cationic aqueous solution. Whitlockite crystals having an average diameter of 44.86 nm were obtained from the sample collected 24 hours after addition of the cationic aqueous solution. The related data is shown in the SEM image of FIG. 10.

### Experimental Example 5. Confirmation of physical properties of prepared whitlockite crystals

Using the method provided by the present application and the conventional method, whitlockite crystals were prepared, and the physical properties of the crystals prepared by each method were confirmed.

### Preparation of whitlockite crystals according to the method provided by the present application

After adding approximately 242 mL of water to a first reactor, 8.58 mL of 85% phosphoric acid (Daejung Chemicals & Metals, Cat. No. 6532-4105 Phosphoric acid, 85%) was added thereto. Subsequently, 250 mL of acidic aqueous solution (0.5M phosphoric acid solution) was prepared by stirring at 80 °C for 1 hour at 250 rpm. After adding 250 mL of water to a second reactor, 6.85 g of calcium hydroxide (Ca(OH)₂) and 1.90 g of magnesium hydroxide (Mg(OH)₂) were added. Afterward, a cationic aqueous solution (250 mL, 0.37M calcium hydroxide, and 0.17M magnesium hydroxide) was prepared by stirring at 60 °C for 1 hour at 250 rpm. The cationic aqueous solution was added to the first reactor containing the acidic aqueous solution at a rate of 2.5 L/min. Here, the solution in the first reactor was stirred at 250 rpm. After the addition of the cationic aqueous solution was completed, the solution in the reactor was aged while stirring for 24 hours at 80 °C and 250 rpm. After the 24-hour aging was completed, the whitlockite crystals were washed to remove ions remaining in the solution. Here, the washing was carried out through a decanting method in which an upper aqueous solution was removed while a sediment settled and water was added (D.W 500 mL x 6EA). The washed resulting product was put into a dry oven and dried at 90 °C for 24 hours, thereby obtaining whitlockite crystals.

### Preparation of whitlockite crystals according to conventional method

After adding 250 mL of water to the first reactor, 6.85 g of calcium hydroxide (Ca(OH)₂) and 1.90 g of magnesium hydroxide (Mg(OH)₂) were added. Subsequently, a cationic aqueous solution was prepared by stirring at 80 °C for 1 hour at 250 rpm. After adding approximately 242 mL of water to a second reactor, 8.58 mL of 85% phosphoric acid (Daejung Chemicals & Metals, Cat. No. 6532-4105 Phosphoric acid, 85%) was added to prepare an acidic aqueous solution. The acidic aqueous solution was added to the cationic aqueous solution included in the first reactor at 12.5 mL/min by a dropwise addition method. Here, the solution in the first reactor was stirred at 250 rpm. After the addition of the acidic aqueous solution was completed, the solution in the reactor was aged while stirring for 24 hours at 80 °C and 250 rpm. After the 24-hour aging, the whitlockite crystals were washed to remove ions remaining in the solution. Here, the washing was carried out through a decanting method in which an upper aqueous solution was removed while a sediment settled and water was added (D.W 500 mL x 6EA). The washed resulting product was put into a dry oven and dried at 90 °C for 24 hours, thereby obtaining whitlockite crystals.

### Results

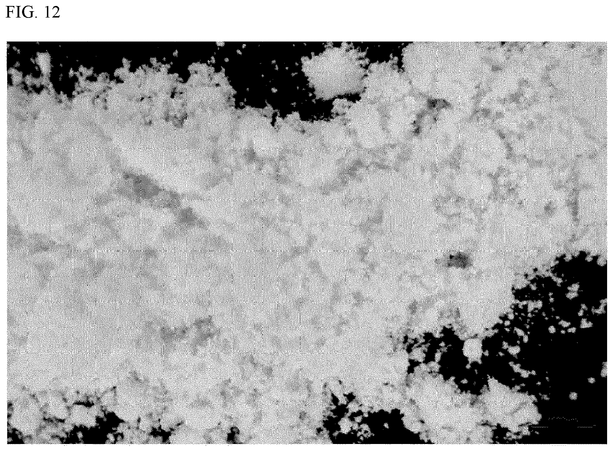

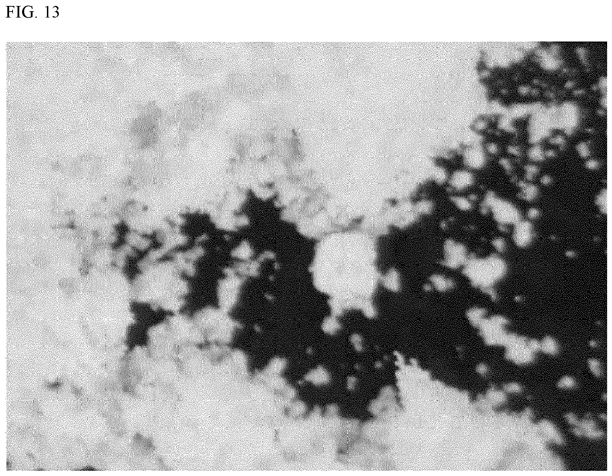

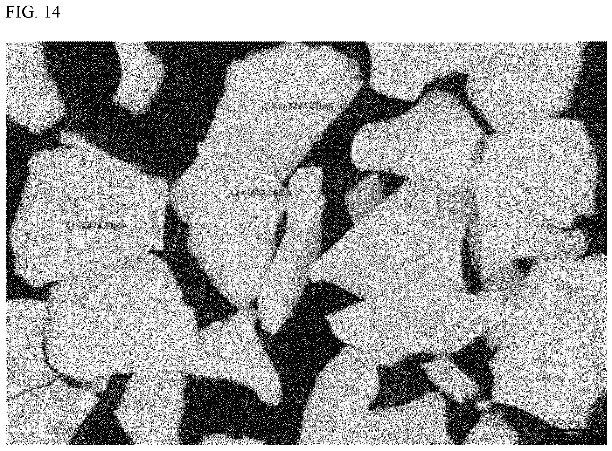

Images of the whitlockite crystals prepared by each method are shown in FIGS. 12 to 14. FIG. 12 is an image of a sample taken with a camera after preparing and then drying the whitlockite crystals according to the method provided by the present application. FIG. 13 is an enlarged image of a part of the image shown in FIG. 12. FIG. 14 is an image of a dried sample taken with a camera after preparing and then drying whitlockite crystals according to the conventional method. FIGS. 12 to 14 show that the whitlockite crystals prepared and dried according to the conventional method are more aggregated than those prepared and dried according to the method provided by the present application. The results show that the sample of whitlockite crystals prepared by the method provided by the present application is more conveniently used and/or processed than those prepared according to the conventional method.

## Claims

1. A method for preparing a whitlockite crystal under acidic condition without forming a hydroxyapatite crystal, comprising:
(i) preparing a first solution containing phosphate ions in a reactor, wherein the first solution is an acidic solution with a pH of 5 or less;
(ii) preparing a second solution containing calcium ions and magnesium ions, wherein the second solution is a basic solution with a pH of 8 or more;
(iii) adding the basic second solution to the reactor comprising the acidic first solution, wherein a mixed solution of the first solution and the second solution is prepared; and
(iv) stirring the solution in the reactor, wherein a pH of the solution in the reactor is 5 or less.

2. The method of claim 1,
wherein, in the (iii), the pH of the solution in the reactor is maintained to 5 or less.

3. The method of claim 1,
wherein, during the entire process of the method, the pH of the solution present in the reactor is 5 or less.

4. The method of claim 1,
wherein the second solution is added for a first time period.

5. The method of claim 4,
wherein the second solution is added at a constant rate, and
wherein the first time period is 120 seconds or less.

6. The method of claim 1,
wherein, in the (iii), the solution in the reactor is stirred.

7. The method of claim 1,
wherein the hydroxyapatite crystal is not produced during the entire process of the method.

8. The method of claim 1,
wherein the hydroxyapatite crystal is not produced during the entire process of the method, and
wherein the whitlockite crystal produced by the method is:
(a) formed directly from the solution in the reactor;
(b) formed by transformation from a crystal comprising a calcium atom other than the hydroxyapatite crystal, formed by transformation from a crystal comprising a magnesium atom other than the hydroxyapatite crystal, or formed by transformation from a crystal comprising the calcium atom and the magnesium atom other than the hydroxyapatite crystal; or
(c) formed by the (a) and the (b).

9. The method of claim 1,
wherein the method further comprises:
drying a result product obtained from the (iv).

10. The method of claim 1,
wherein the method further comprises:
(v) washing a result product obtained from the (iv); and
(vi) drying a result product obtained from the (v).

11. The method of claim 1,
wherein an amount of phosphorus atoms which the mixed solution contains is a first amount, an amount of calcium atoms which the mixed solution contains is a second amount, and an amount of magnesium atoms which contains the mixed solution is a third amount,
wherein any one or more selected from a ratio between the first amount and the second amount, a ratio between the second amount and the third amount, a ratio between the first amount and the third amount are determined by considering one or more of the follows:
the ratio of the phosphorus atoms, the calcium atoms, and the magnesium atoms (P: Ca: Mg) constituting whitlockite;
the pH of the mixed solution is 5 or less; and
90wt% or more of solids produced through the method is whitlockite crystals.

12. The method of claim 11,
wherein the ratio of the phosphorus atoms and the calcium atoms constituting whitlockite is 0.78,
wherein the ratio of the first amount which is the amount of the phosphorus atoms which the mixed solution contains and the second amount which is the amount of the calcium atoms which the mixed solution contains (P/Ca) is greater than 0.78.

13. The method of claim 1,
wherein a ratio of a molar quantity of the calcium atoms which the mixed solution contains and a molar quantity of the magnesium atoms which the mixed solution contains is equal or more than 2.2 and equal or less than 4.0.
